# EUROPÄISCHE PATENTSCHRIFT

(11) **EP 2 753 290 B1**
(45) Veröffentlichungstag und Bekanntmachung des Hinweises auf die Patenterteilung: **12.06.2019**
(21) Anmeldenummer: 12761572.2
(22) Anmeldetag: 10.09.2012
(51) Int. Cl.: A61K 6/00, A61K 6/083

(54) **DENTALMATERIALIEN AUF BASIS VON MONOMEREN MIT DEBONDING-ON-DEMAND EIGENSCHAFTEN**
DENTAL MATERIALS ON THE BASIS OF MONOMERS WITH DEBONDING ON DEMAND PROPERTIES
MATÉRIAUX DENTAIRES À BASE DE MONOMÈRES AYANT DES PROPRIÉTÉS DE DÉCOLLEMENT À LA DEMANDE

(30) Priorität: 08.09.2011 EP 11180645
(43) Veröffentlichungstag der Anmeldung: 16.07.2014
(73) Patentinhaber: Ivoclar Vivadent AG, 9494 Schaan (LI)
(72) Erfinder: MOSZNER, Norbert, CH-9495 Triesen (LI); LAMPARTH, Iris, CH-9472 Grabs (CH); BOCK, Thorsten, A-6800 Feldkirch (AT); FISCHER, Urs, Karl, CH-9320 Arbon (CH); SALZ, Ulrich, 88131 Lindau (DE); RHEINBERGER, Volker, CH-9490 Vaduz (LI); LISKA, Robert, A-2123 Schleinbach (AT)
(74) Vertreter: Uexküll & Stolberg
(86) Internationale Anmeldenummer: PCT/EP2012/067679
(87) Internationale Veröffentlichungsnummer: WO 2013/034777

(56) Entgegenhaltungen:
- WO-A1-2008/005173
- US-A1- 2007 142 494
- US-A1- 2007 142 497

## Beschreibung

Die vorliegende Erfindung betrifft Monomere mit Debonding-on-Demand (DoD) Eigenschaften und deren Verwendung als Monomerkomponenten in Dentalmaterialien, insbesondere zur Herstellung von Adhäsiven und Zementen.

Wiederlösbare Klebeverbindungen haben in verschiedenen Bereichen der Technik eine zunehmende Bedeutung. Beispiele sind das Entfügen von Bauteilen im Rahmen von automatisierten Fertigungsprozessen, die Reparatur von komplexen Bauteilen mit geklebten Teilkomponenten oder die Vereinfachung der stofflichen Trennung beim Recycling solcher Bauteile am Produktlebensende. Das Lösen (debonding) von Klebeverbindungen kann gezielt (on demand) dadurch erreicht werden, dass die Festigkeit der adhäsiven Verbundschicht z.B. durch Erwärmung deutlich verringert wird.

So beschreibt DE 198 32 629 A1 ein Klebstoffsystem zur Bildung reversibler Klebeverbindungen auf Basis von Polyurethanen, Polyharnstoffen oder Epoxidharzen, bei der eine Zusatzkomponente durch Energieeintrag so aktiviert werden kann, dass ein Abbau der Klebstoffkomponenten erfolgt. Beispielsweise können durch Eintrag von Wärme- oder Strahlungsenergie aus blockierten Precursoren organische Basen oder Säuren freigesetzt werden, die einen Abbau des Kleberharzes bewirken.

WO 2010/128042 A1 beschreibt technische Klebstoffzusammensetzungen für lösbare Klebeverbindungen für den Flugzeug- oder Fahrzeugbau, die aus einer üblichen Klebstoffmatrix und einem partikelförmigen Expansionsstoff wie z.B. Azodicarbonamid bestehen. Dabei erfolgt das Lösen der Bauteile durch Erwärmen der Klebeverbindung mindestens auf die Expansionstemperatur des Expansionsstoffes.

Im Dentalbereich ist das Lösen von Klebeverbindungen unter anderem in der Kieferorthopädie von Bedeutung, wo Brackets, die zur Korrektur von Zahnfehlstellungen auf die Zahnoberfläche geklebt werden, nach erfolgter Korrektur ohne Schädigung des Zahnschmelz wieder entfernen werden müssen. Ausserdem wären im Falle der Reparatur oder des vollkommenen Ersatzes von hochfesten keramischen Restaurationen oder Kronen, die mechanisch nur aufwändig entfernbar sind, einfach erweichbare bzw. trennbare Zementverbunde von Vorteil.

Im Zusammenhang mit orthodontischen Anwendungen beschreibt US 2007/0142498 A1 dentale Zusammensetzungen, die thermisch steuerbare Additive wie z.B. thermoplastische Polymere enthalten.

US 2007/0142497 A1 beschreibt dentale Zusammensetzungen auf Basis von Dimethacrylaten mit säurelabilen tertiären Carbonatgruppen und Photosäuren wie z.B. Triarylsulfoniumsalzen. Diese Zusammensetzungen lassen sich mit geeigneten Initiatoren wie etwa dem Bisacylphosphinoxid Irgacure 819 mit Licht im sichtbaren Bereich photochemisch härten (Photobonding) und durch Bestrahlung mit UV-Licht bei erhöhter Temperatur wieder erweichen (photothermisches Debonding).

US 2007/0142494 A1 beschreibt dentale Zusammensetzungen, die Komponenten mit thermolabilen Gruppen enthalten, wobei es sich bei den thermolabilen Gruppen unter anderem um Cycloadditionsaddukte wie Diels-Alder-Addukte handeln kann. Als Beispiel wird eine dimere Verbindung beschrieben, in der zwei Diels-Alder-Gruppen über Arylreste als Linker-Gruppen verbrückt sind.

Der Erfindung liegt die Aufgabe zugrunde, adhäsive Dentalwerkstoffe bereitzustellen, die polymerisierbar sind, gute Substrathaftung insbesondere auf Zahnhartsubstanz und/oder Dentalkeramiken zeigen und durch Wärmezufuhr ein Debonding vom Substrat gestatten und sich damit vor allem zur Herstellung von Adhäsiven oder Kompositzementen mit Debonding-on-Demand-Eigenschaften eignen.

Diese Aufgabe wird erfindungsgemäss durch Dentalwerkstoffe auf Basis einer thermolabilen polymerisierbaren Verbindung der Formel II gelöst: 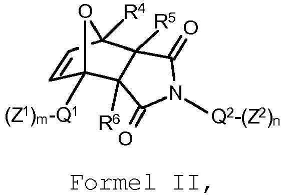 in der
- Z¹ und Z²: jeweils unabhängig für eine polymerisierbare Gruppe ausgewählt aus Vinylgruppen, CH₂=CR¹-CO-O- und CH₂=CR¹-CO-NR²- oder für eine adhäsive Gruppe ausgewählt aus -Si(OR)₃, -COOH, -O-PO(OH)₂, -PO(OH)₂, -SO₂OH und -SH stehen, wobei mindestens ein Z¹ oder Z² eine polymerisierbare Gruppe ist,
- Q¹: jeweils unabhängig entfällt oder für einen (m+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³- unterbrochen sein kann,
- Q²: jeweils unabhängig entfällt oder für einen (n+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³- unterbrochen sein kann,
- R, R¹, R² und R³: jeweils unabhängig für H oder einen C₁-C₇-Alkyl-Rest stehen,
- R⁴: H oder ein C₁-C₁₀-Alkyl-Rest ist,
- R⁵: H, ein C₁-C₅-Alkyl-Rest, F oder CN ist,
- R⁶: H, ein C₁-C₅-Alkyl-Rest, F oder CN ist und
- m und n: jeweils unabhängig 1, 2 oder 3 sind.

Die Formel II umfasst dabei erfindungsgemäss sowohl reine *exo-*Produkte bzw. reine *endo*-Produkte als auch Mischungen von *exo-* und *endo*-Produkten.

In einer Ausführungsform ist mindestens ein Z¹ oder Z² eine polymerisierbare Gruppe und mindestens ein Z¹ oder Z² eine adhäsive Gruppe. Dabei sind solche Verbindungen der Formel II bevorzugt, bei denen eines von Z¹ und Z² für eine polymerisierbare Gruppe und das andere von Z¹ und Z² für eine adhäsive Gruppe steht. In einer anderen Ausführungsform stehen Z¹ und Z² beide für eine polymerisierbare Gruppe.

Der Hinweis, dass ein Rest durch eine Gruppe wie beispielsweise -O- unterbrochen sein kann, ist so zu verstehen, dass die Gruppe in die Kohlenstoffkette des Restes eingeschoben wird, d.h. beidseitig von Kohlenstoffatomen begrenzt wird. Die Anzahl dieser Gruppen ist daher um mindestens 1 kleiner als die Zahl der Kohlenstoffatome, und die Gruppen können nicht endständig sein. Erfindungsgemäss sind Reste, die nicht durch die genannten Gruppen unterbrochen sind, bevorzugt.

Erfindungsgemäss werden nur solche Verbindungen in Erwägung gezogen, die in mit der chemischen Valenzlehre vereinbar sind.

Besonders bevorzugt sind solche Verbindungen der Formel II, bei denen jeweils unabhängig voneinander
- eines von Z¹ und Z²: jeweils unabhängig für eine polymerisierbare Gruppe ausgewählt aus CH₂=CR¹-CO-O- und CH₂=CR¹-CO-NR²- steht und das andere von Z¹ und Z² jeweils unabhängig für eine polymerisierbare Gruppe ausgewählt aus CH₂=CR¹-CO-O- und CH₂=CR¹-CO-NR²- oder vorzugsweise für eine adhäsive Gruppe ausgewählt aus -Si(OR)₃, -COOH, -O-PO(OH)₂, -PO(OH)₂, -SO₂OH und -SH steht,
- Q¹: jeweils unabhängig entfällt oder für einen (m+1)-wertigen linearen oder verzweigten aliphatischen C₁-C₁₀-Rest steht, der durch -O-, -CO-O-, -O-CO-, -CO-NR³- oder -NR³-CO- unterbrochen sein kann,
- Q²: jeweils unabhängig entfällt oder für einen (n+1)-wertigen linearen oder verzweigten aliphatischen C₁-C₁₀-Rest steht, der durch -O-, -CO-O-, -O-CO-, -CO-NR³- oder -NR³-CO- unterbrochen sein kann,
- R: jeweils unabhängig H, CH₃ oder C₂H₅ ist,
- R¹: jeweils unabhängig H oder CH₃ ist,
- R²: jeweils unabhängig H, CH₃ oder C₂H₅ ist,
- R³: jeweils unabhängig H, CH₃ oder C₂H₅ ist und/oder
- m und n: jeweils unabhängig 1 oder 2 sind.

Besonders bevorzugt sind Verbindungen, in denen alle Variablen jeweils eine der oben definierten bevorzugten Bedeutungen haben.

In einer bevorzugten Ausführungsform sind solche Verbindungen der Formel II bevorzugt, bei denen
- Z¹ und Z²: jeweils unabhängig für eine polymerisierbare Gruppe ausgewählt aus Vinylgruppen, CH₂=CR¹-CO-O- und CH₂=CR¹-CO-NR²- oder für eine acide Gruppe ausgewählt aus -O-PO(OH)₂, -PO(OH)₂ und -SO₂OH stehen, wobei mindestens ein Z¹ oder Z² eine polymerisierbare Gruppe und mindestens ein Z¹ oder Z² eine acide Gruppe ist,
- Q¹: jeweils unabhängig entfällt oder für einen (m+1)-wertigen linearen oder verzweigten aliphatischen C₁-C₁₀-Rest steht, der durch -O-, -S-, -CO-O-, -O-CO-, -CO-NR³- oder -NR³-CO-unterbrochen sein kann,
- Q²: jeweils unabhängig entfällt oder für einen (n+1)-wertigen linearen oder verzweigten aliphatischen C₁-C₁₀-Rest steht, der durch -O-, -S-, -CO-O-, -O-CO-, -CO-NR³- oder -NR³-CO-unterbrochen sein kann,
- R¹, R² und R³: jeweils unabhängig für H oder einen C₁-C₇-Alkyl-Rest stehen und
- m und n: jeweils unabhängig 1, 2 oder 3 sind.

Dabei sind solche Verbindungen der Formel II bevorzugt, bei denen eines von Z¹ und Z² für eine polymerisierbare Gruppe und das andere von Z¹ und Z² für eine acide Gruppe steht.

Besonders bevorzugt sind dabei solche Verbindungen der Formel II, bei denen jeweils unabhängig voneinander
- eines von Z¹ und Z²: jeweils unabhängig für eine polymerisierbare Gruppe ausgewählt aus CH₂=CR¹-CO-O- und CH₂=CR¹-CO-NR²- steht und das andere von Z¹ und Z² jeweils unabhängig für eine acide Gruppe ausgewählt aus -O-PO(OH)₂ und -PO(OH)₂ und-SO₂OH steht,
- Q¹: jeweils unabhängig entfällt oder für einen (m+1)-wertigen linearen oder verzweigten aliphatischen C₁-C₁₀-Rest steht, der durch -O-, -CO-O-, -O-CO-, -CO-NR³- oder -NR³-CO- unter-brochen sein kann,
- Q²: jeweils unabhängig entfällt oder für einen (n+1)-wertigen linearen oder verzweigten aliphatischen C₁-C₁₀-Rest steht, der durch -O-, -CO-O-, -O-CO-, -CO-NR³- oder -NR³-CO- unter-brochen sein kann,
- R¹: jeweils unabhängig H oder CH₃ ist,
- R²: jeweils unabhängig H, CH₃ oder C₂H₅ ist,
- R³: jeweils unabhängig H, CH₃ oder C₂H₅ ist und/oder
- m und n: jeweils unabhängig 1 oder 2 sind.

Besonders bevorzugt sind Verbindungen, in denen alle Variablen jeweils eine der oben definierten bevorzugten Bedeutungen haben.

Weiterhin sind solche Verbindungen der Formel II bevorzugt, bei denen jeweils unabhängig voneinander
- eines von Z¹ und Z²: jeweils unabhängig für eine polymerisierbare Gruppe ausgewählt aus CH₂=CR¹-CO-O- und CH₂=CR¹-CO-NR²- steht und das andere von Z¹ und Z² jeweils unabhängig für eine polymerisierbare Gruppe ausgewählt aus CH₂=CR¹-CO-O- und CH₂=CR¹-CO-NR²- oder vorzugsweise für eine adhäsive Gruppe ausgewählt aus -Si(OR)₃, -COOH, -O-PO(OH)₂, -PO(OH)₂, -SO₂OH und -SH steht,
- Q¹: jeweils unabhängig entfällt oder für einen (m+1)-wertigen linearen oder verzweigten aliphatischen C₁-C₁₅-Rest, vorzugsweise einen C₁-C₁₀-Rest, bevorzugt einen C₁-C₈-Rest, insbesondere einen C₂-C₆-Rest und besonders bevorzugt einen C₁-C₂-Rest steht, der durch -O-, -CO-O-, -O-CO-, -CO-NR³-, -NR³-CO-, -O-CO-NR³-, -NR³-CO-O- oder -NR³-CO-NR³- unterbrochen sein kann,
- Q²: jeweils unabhängig entfällt oder für einen (n+1)-wertigen linearen oder verzweigten aliphatischen C₁-C₁₅-Rest, vorzugsweise einen C₁-C₁₀-Rest, bevorzugt einen C₁-C₈-Rest, insbesondere einen C₂-C₆-Rest und besonders bevorzugt einen C₂-C₃-Rest steht, der durch -O-, -CO-O-, -O-CO-, -CO-NR³-, -NR³-CO-, -O-CO-NR³-, -NR³-CO-O- oder -NR³-CO-NR³- unterbrochen sein kann,
- R: jeweils unabhängig CH₃ oder C₂H₅ ist,
- R¹: jeweils unabhängig H oder CH₃ ist,
- R²: jeweils unabhängig H, CH₃ oder C₂H₅ ist,
- R³: jeweils unabhängig H, CH₃ oder C₂H₅ ist,
- R⁴: H, CH₃ oder C₂H₅ ist,
- R⁵: H, F oder CN und insbesondere H ist,
- R⁶: H, F oder CN und insbesondere H ist und/oder
- m und n: jeweils unabhängig 1 oder 2 sind.

Ganz besonders bevorzugt sind Verbindungen der Formel II, bei denen jeweils unabhängig voneinander
- eines von Z¹ und Z²: jeweils unabhängig für eine polymerisierbare Gruppe ausgewählt aus CH₂=CR¹-CO-O- und CH₂=CR¹-CO-NR²- steht und das andere von Z¹ und Z² jeweils unabhängig für eine polymerisierbare Gruppe ausgewählt aus CH₂=CR¹-CO-O- und CH₂=CR¹-CO-NR²- oder vorzugsweise für eine adhäsive Gruppe ausgewählt aus -Si(OR)₃, -O-PO(OH)₂, -PO(OH)₂ und -SH steht,
- Q¹: für einen Methylen- oder Ethylen-Rest steht,
- Q²: für einen Ethylen- oder Propylen-Rest steht,
- R: jeweils unabhängig CH₃ oder C₂H₅ ist,
- R¹: jeweils unabhängig H oder CH₃ ist,
- R²: jeweils unabhängig H, CH₃ oder C₂H₅ ist,
- R³: jeweils unabhängig H, CH₃ oder C₂H₅ ist,
- R⁴: H, CH₃ oder C₂H₅ ist,
- R⁵: H, F oder CN und insbesondere H ist,
- R⁶: H, F oder CN und insbesondere H ist und/oder
- m und n: jeweils 1 sind.

Besonders bevorzugt sind Verbindungen, in denen alle Variablen jeweils eine der oben definierten bevorzugten Bedeutungen haben.

Es wurde überraschend gefunden, dass die erfindungsgemässen Dentalwerkstoffe, die mindestens eine thermolabile polymerisierbare Verbindung der Formel II enthalten, nach der Polymerisation einerseits hervorragende mechanische Eigenschaften sowie eine hervorragende Haftung auf Zahnhartsubstanz und Dentalkeramiken zeigen und sich andererseits durch Wärmezufuhr (thermolabile Verbindungen) leicht vom Substrat lösen lassen.

Die polymerisierbaren Diels-Alder-Addukte der Formel II lassen sich einfach herstellen. Beispielsweise lassen sich geeignet funktionalisierte Furan-Derivate mit geeignet N-funktionalisierten Maleinimiden unter für Diels-Alder-Reaktion üblichen Reaktionsbedingungen und insbesondere bei 80-120 °C z.B. in aromatischen Lösungsmitteln und gegebenenfalls unter Zusatz eines geeigneten Katalysators (beispielsweise Brönsted- oder Lewis-Säuren) sowie eines Polymerisationsinhibitors (vgl. Autorenkollektiv, Organikum, Wiley-VCH, 21. Aufl., Weinheim etc. 2001, 330 ff.) zu einem entsprechenden polymerisierbaren Diels-Alder-Addukt umsetzen:

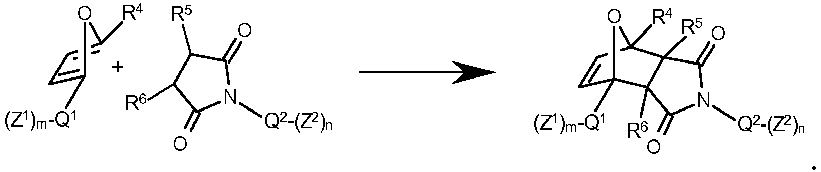

### Konkretes Beispiel: Diels-Alder-Reaktion von Furfurylmethacrylat (Z¹ = CH₂=CR¹-CO-O-, Q¹ = -CH₂-, R¹ = CH₃, R⁴ = H und m = 1) und N-[3-(Dihydroxyphosphoryl)propyl]-maleinimid (Z² = -PO(OH)₂, Q² =-(CH₂)₃-, R⁵ und R⁶ = H und n = 1) :

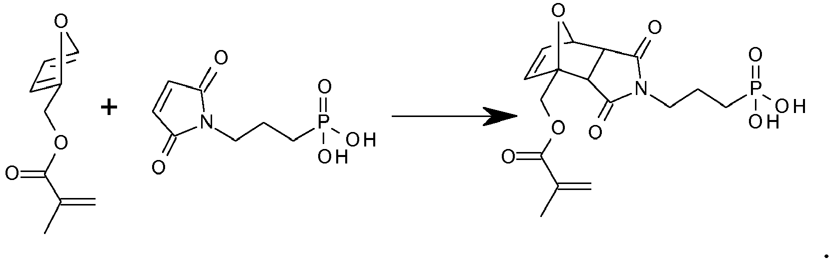

Geeignete Ausgangsstoffe für die Synthese von mit polymerisierbaren oder stark aciden Gruppen funktionalisierten Furan-Derivaten sind kommerziell zugänglich, beispielsweise Furfural, Furfurylalkohol oder Brenzschleimsäure (vgl. Ullmann's Encyclopedia of Industrial Chemistry, 5th Ed., Vol. A 12, VCH, Weinheim etc. 1989, Seite 119 ff.). Substituierte Furanderivate lassen sich beispielsweise durch Paal-Knorr-Synthese durch Erhitzen von entsprechenden 1,4-Diketoverbindungen herstellen (vgl. W. Walter, W. Francke, Beyer-Walter Lehrbuch der Organischen Chemie, S. Hirzel Verlag, Stuttgart und Leipzig 2004, 24. Aufl., Seite 769). Mit polymerisierbaren oder stark aciden Gruppen funktionalisierte Maleinimide lassen sich am einfachsten durch Umsetzung von Maleinsäureanhydrid mit entsprechend funktionalisierten Aminen herstellen.

Bei der Synthese der Diels-Alder-Addukte der Formel II kann auch schrittweise so vorgegangen werden, dass zunächst ein geeignetes Diels-Alder-Addukt aus einem geeignet funktionalisierten Maleinimid und Furanderivat hergestellt wird und erst danach die Einführung der polymerisierbaren bzw. der stark aciden Säuregruppen erfolgt, wobei die Synthesen gegebenenfalls unter Verwendung von Schutzgruppen durchgeführt werden. Beispielsweise kann das oben beispielhaft angeführte Diels-Alder-Addukt mit einer polymerisierbaren Methacrylat- und einer stark aciden PhosphonsäureGruppe auch so hergestellt werden, dass zunächst der z.B. mit einer Tetrahydropyranyl (THP)-Gruppe geschützte Furfurylalkohol mit N-(3-Brompropyl)maleinimid zum Diels-Alder-Addukt umgesetzt wird. Nach Einführung der Phosphonsäuregruppe z.B. über eine Michaelis-Arbusow-Reaktion durch Umsetzung des Diels-Alder-Addukts etwa mit Triethylphosphit (P(OC₂H₅)₃) kann die THP-Schutzgruppe wieder abgespalten, die gebildete OH-Gruppe z.B. mit Methacrylsäureanhydrid (MAAH) methacryliert und schliesslich die Phosphonsäuregruppe hydrolytisch freigesetzt werden:

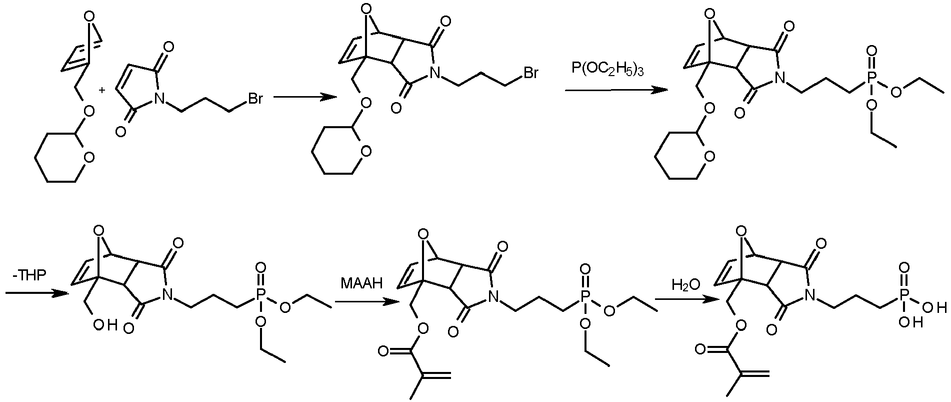

Beispiele für erfindungsgemässe thermolabile Diels-Alder-Addukte der Formel II sind:

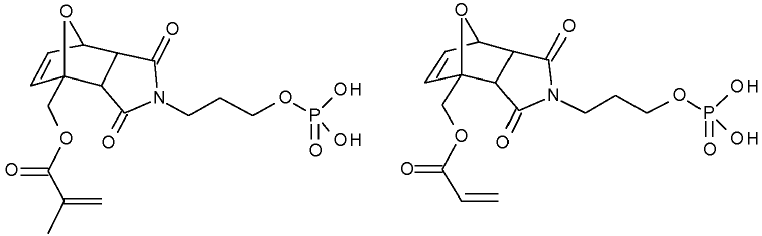

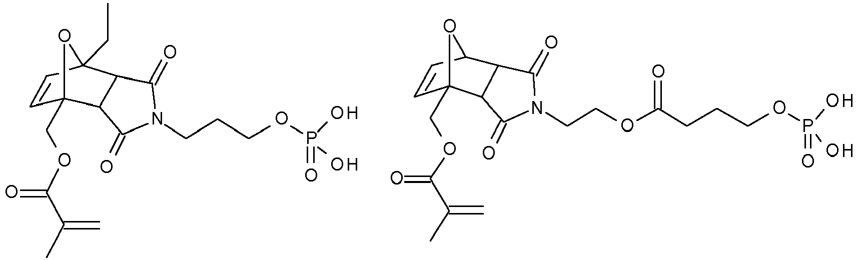

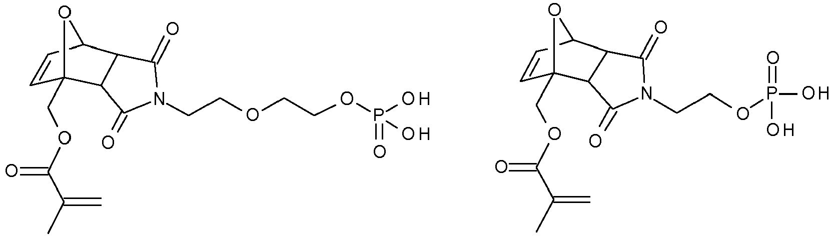

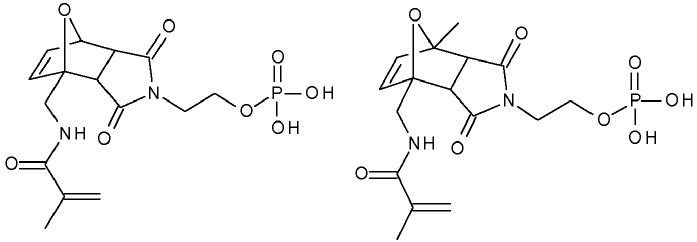

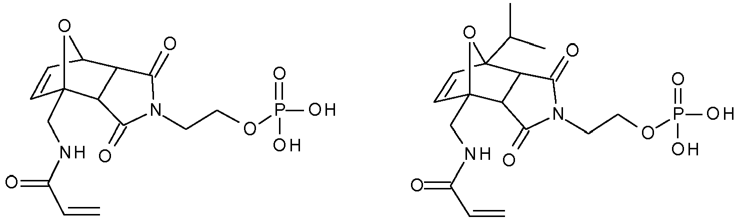

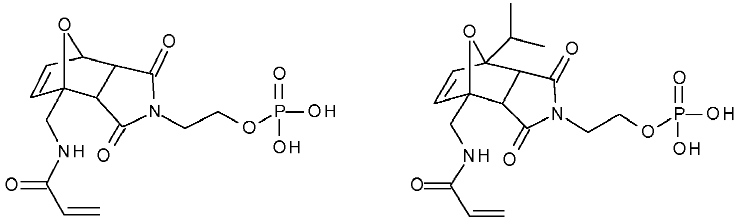

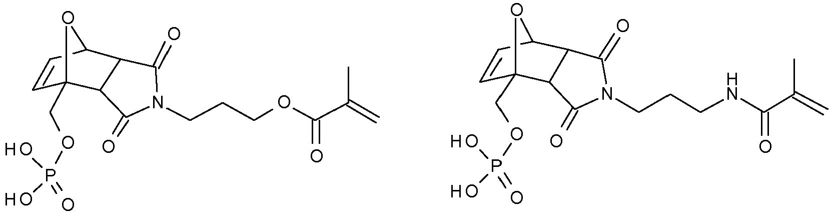

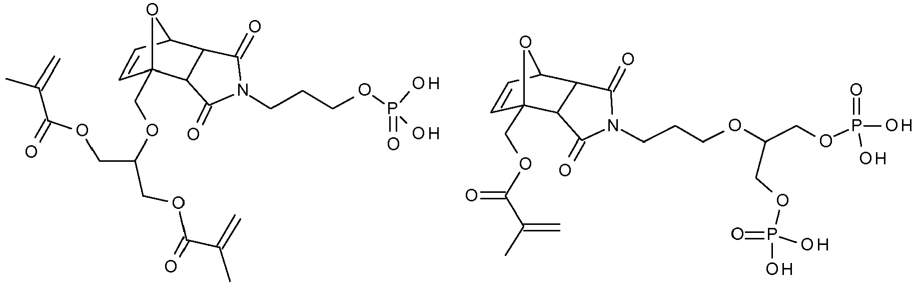

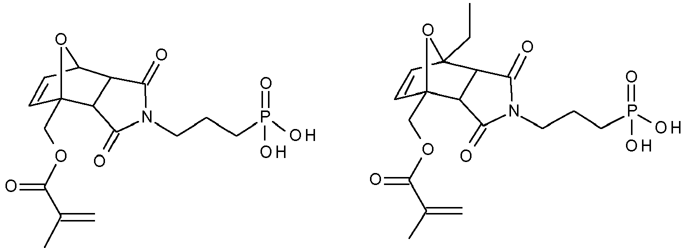

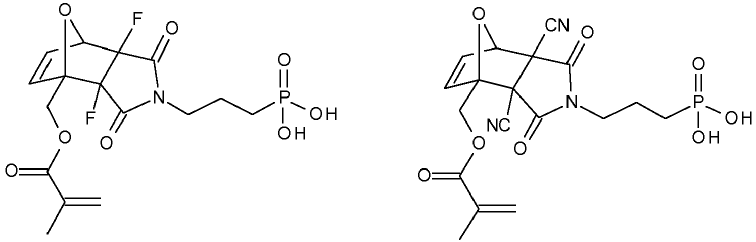

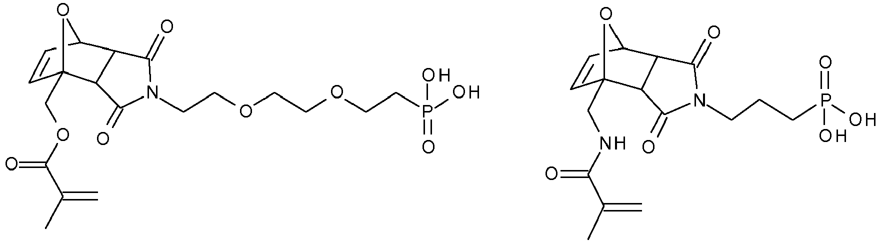

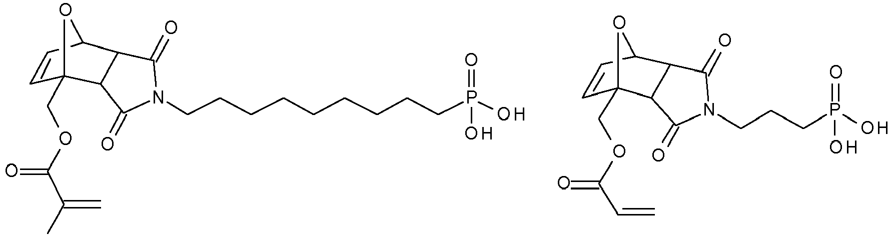

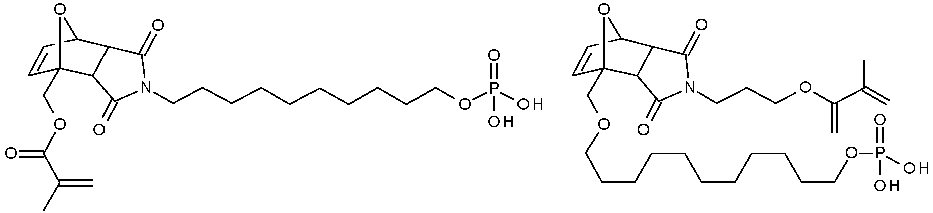

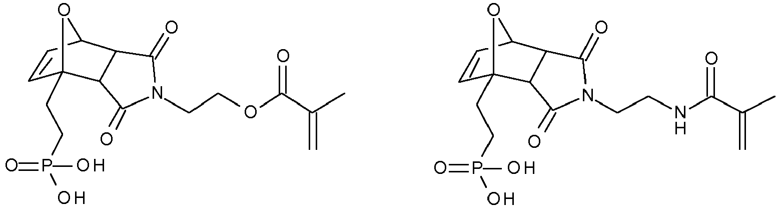

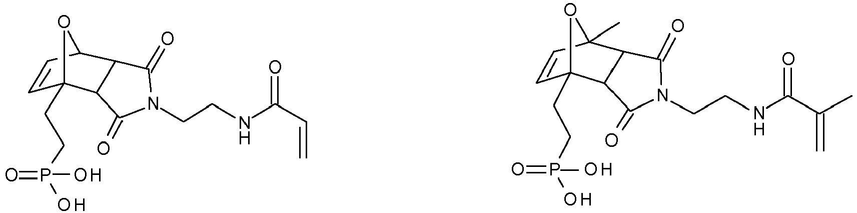

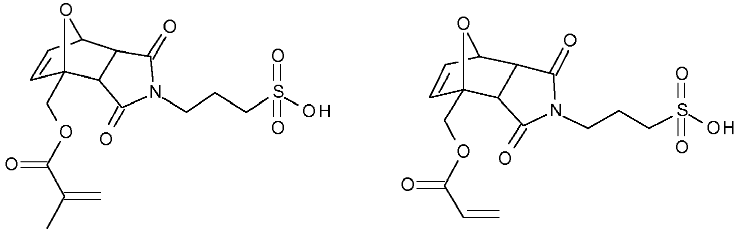

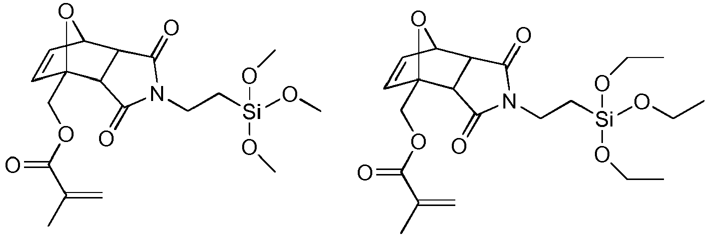

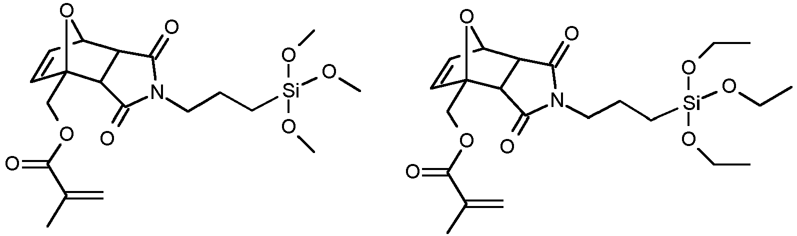

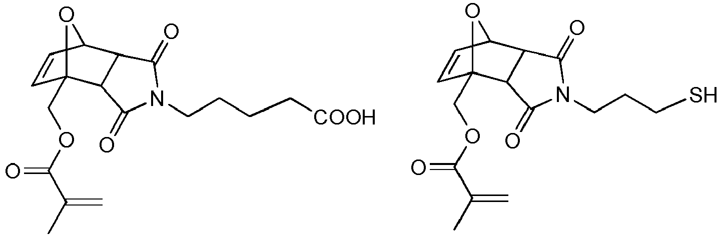

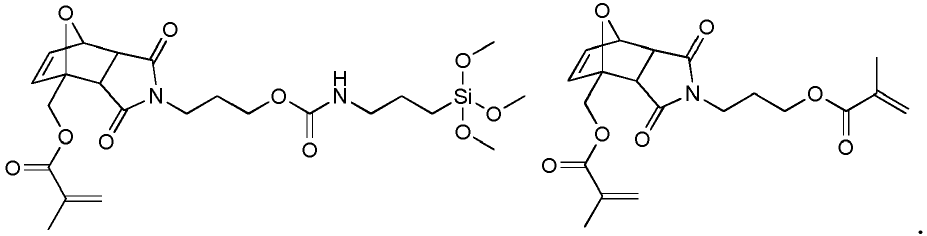

Die erfindungsgemässen Dentalmaterialien enthalten neben der thermolabilen polymerisierbaren Verbindung der Formel II vorzugsweise ein oder mehrere zusätzliche radikalisch polymerisierbare Monomere (Co-Monomere), insbesondere mono- oder polyfunktionelle (Meth)acrylsäurederivate. Unter monofunktionellen (Meth)acrylsäurederivaten werden Verbindungen mit einer, unter polyfunktionellen (Meth)acrylsäurederivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäss bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi(meth)acrylat, 1,4-Butandioldi(meth)acrylat, 1,10-Decandioldi(meth)acrylat und 1,12-Dodecandioldi(meth)acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)-methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis(3-vinyl-2-pyrrolidonyl)-hexan, Bisacrylamide wie Methylen- oder Ethylenbisacrylamid und Bis(meth)acrylamide wie N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin, die durch Umsetzung aus den entsprechenden Diaminen mit (Meth)acrylsäurechlorid synthetisiert werden können.

Erfindungsgemäss als Co-Monomere besonders geeignet sind auch thermolabile Vernetzermonomere. Thermolabile Vernetzermonomere weisen mindestens eine thermolabile Gruppe zwischen zwei polymerisierbaren Gruppen auf. Beispiele sind polyfunktionelle (Meth)acrylate oder (Meth)acrylamide mit mindestens einer thermolabilen Gruppe zwischen zwei (Meth)acrylgruppen. Dabei kommen als thermolabile Gruppen grundsätzlich dieselben Gruppen in Frage, wie sie oben für die Verbindungen der Formel II definiert sind und insbesondere thermolabile Cycloadditionsaddukte wie Diels-Alder-Addukte, Hetero-Diels-Alder-Addukte sowie thermolabile Alkoxyamin-, Oximester-, Oximurethan- oder Azogruppen. Beispiele sind Diels-Alder-Addukte wie das Diels-Alder-Addukt aus Furfurylmethacrylat und N-(3-(Methacryloyloxy)propyl)-maleinimid, die Umsetzungsprodukte von N-Hydroxy-(meth)acrylamid mit Di- oder Triisocyanaten wie Hexamethylen-1,6-diisocyanat (HDI), 2,2,4-Trimethylhexamethylen-1,6-diisocyanat oder dem HDI-Trimer sowie Produkte, die durch stöchiometrische Umsetzung von Di- oder Triisocyanaten mit 1-Hydroxymethylacrylsäureestern wie 1-Hydroxymethylacrylsäureethylester oder mit β-Ketoester(meth)acrylaten wie 2-Acetoacetoxyethylmethacrylat erhalten werden. Besonders geeignet sind auch gasfreisetzende thermolabile Vernetzermonomere. Beispiele sind die Veresterungsprodukte von Azobis(4-cyanovaleriansäure) mit Hydroxyalkyl(meth)acrylaten wie Hydroxyethyl(meth)acrylat oder Hydroxypropyl(meth)acrylat oder mit N-(Hydroxyalkyl)(meth) acrylamiden wie N-(5-Hydroxypentyl)methacrylamid oder N-Methyl-N-(2-hydroxyethyl)acrylamid.

Die erfindungsgemässen Dentalwerkstoffe können neben der thermolabilen polymerisierbaren Verbindung der Formel
II und ggf. den oben genannten Co-Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisierbaren Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Monomere mit polymerisierbaren Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester.

Bevorzugte Monomere mit polymerisierbaren Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propylamino)-propan-2-yl-dihydrogenphosphat.

Bevorzugte Monomere mit polymerisierbaren Sulfonsäuregruppen sind Vinylsulfonsäure, 4-Vinylphenylsulfonsäure und 3-(Methacrylamido)-propylsulfonsäure.

Vorzugsweise werden Mischungen der vorstehend genannten Monomere verwendet. Bevorzugte Monomermischungen enthalten bezogen auf das Gesamtgewicht der Monomermischung:
1 bis 90 Gew.-%, vorzugsweise 5 bis 80 Gew.-%, besonders bevorzugt 5 bis 70 Verbindung der Formel II,
0 bis 70 Gew.-%, vorzugsweise 1 bis 60 Gew.-%, besonders bevorzugt 5 bis 50 und ganz besonders bevorzugt 10 bis 30 Gew.-% Co-Monomer und insbesondere mono- und/oder polyfunktionelle (Meth)acrylate,
0 bis 70 Gew.-%, vorzugsweise 1 bis 60 Gew.-% und besonders bevorzugt 5 bis 50 Gew.-% thermolabiles Vernetzermonomer und 0 bis 40 Gew.-%, vorzugsweise 1 bis 30 Gew.-% und besonders bevorzugt 5 bis 20 Gew.-% Haftmonomer.

Besonders bevorzugte Monomermischungen (jeweils bezogen auf das Gesamtgewicht der Monomermischung) sind in der nachfolgenden Tabelle wiedergegeben:

| Komponente (Gew.-%) | A | B | C | D | E | F |
|---|---|---|---|---|---|---|
| Verbindung der der Formel II | 1-90 | 5-80 | 5-70 | 5-70 | 5-70 | 5-70 |
| Co-Monomer, insbesondere mono- und/oder polyfunktionelles (Meth)acrylat | 0-70 | 0-60 | 1-60 | 5-60 | 5-50 | 0-30 |
| thermolabiles Vernetzermonomer | 0-70 | 0-50 | 0-50 | 5-50 | 5-50 | 5-50 |
| Haftmonomer | 0-40 | 0-30 | 0-30 | 0-20 | 0-20 | 0-30 |

Ausserdem enthalten die erfindungsgemässen Dentalwerkstoffe vorzugsweise auch einen Initiator für die radikalische Polymerisation.

Zur Initiierung der radikalischen Photopolymerisation werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4'-Dichlorbenzil eingesetzt. Besonders bevorzugt werden Campherchinon und 2,2-Dimethoxy-2-phenyl-acetophenon und ganz besonders bevorzugt α-Diketone in Kombination mit Aminen wie 4-(Dimethylamino)-benzoesäureester, N,N-Dimethylaminoethylmethacrylat, N,N-Dimethyl-sym.-xylidin oder Triethanolamin als Reduktionsmittel verwendet. Besonders geeignet sind auch Norrish-Typ-I-Photoinitiatoren, insbesondere Acyl- oder Bisacylphosphinoxide, Monoacyltrialkyl- oder Diacyldialkylgermanium-Verbindungen wie Benzoyltrimethylgermanium, Dibenzoyldiethylgermanium oder Bis-(4-methoxybenzoyl)diethylgermanium. Dabei lassen sich auch Mischungen der verschiedenen Photoinitiatoren wie beispielsweise Dibenzoyldiethylgermanium in Kombination mit Campherchinon und 4-Dimethylaminobenzoesäureethylester einsetzen.

Als Initiatoren für eine bei Raumtemperatur durchgeführte Polymerisation werden vorzugsweise Redox-Initiatorkombinationen wie beispielsweise Kombinationen von Benzoylperoxid mit N,N-Dimethylsym.-xylidin oder N,N-Dimethyl-p-toluidin verwendet. Darüber hinaus sind auch Redoxsysteme bestehend aus Peroxiden und solchen Reduktionsmitteln wie z.B. Ascorbinsäure, Barbituraten oder Sulfinsäuren besonders geeignet.

Die erfindungsgemässen Dentalwerkstoffe können zudem ein thermisch gasfreisetzendes Additiv enthalten. Geeignete gasfreisetzende Additive sind z.B. Azo-Verbindungen wie Azodicarbonamid, 2,2'-Azobisisobutyronitril oder 2,2'-Azobis(4-cyano-pentansäure), N-Nitrosoverbindungen, Hydrazide wie Benzolsulfonylhydrazid, Peroxide wie Dicumolperoxid oder Acetondicarbonsäure. Beispiele für solche Verbindungen sind etwa in St. Quinn, Plastics, Additives & Compounding 2001, 3, 16-21 beschrieben. Dabei kann die Zerfallstemperatur beispielsweise im Falle der Azoverbindungen in an sich bekannter Weise durch das Substituentenmuster eingestellt werden (vgl. D. Braun, R. Jakobi, Monatshefte Chemie 1982, 113, 1403-1414).

Weiterhin können die erfindungsgemässen Dentalwerkstoffe ein Additiv enthalten, das eingestrahlte elektromagnetische Strahlung in Wärme umwandeln kann. Solche sogenannten Strahlung-Wärme-Umwandler sind organische, anorganische oder metallorganische Stoffe oder Hybridkomponenten, die in der Lage sind, UV-, NIR- oder IR-Strahlung, sichtbares Licht, Mikrowellen- oder Radiowellenstrahlung in Wärme umzuwandeln, um thermolabile Gruppen zu spalten. Beispiele hierfür sind UV-, NIR oder IR-Strahlung absorbierende Farbstoffe und Pigmente. Beispiele für im IR-Bereich absorbierende Farbstoffe sind Azo-, Methin-, Anthrachinon oder Porphyrinfarbstoffe. Beispiele für NIR-Strahlung absorbierende Pigmente sind Antimon- und Indiumzinnoxid, Phthalocyaninpigmente, Russ, Ni- und Pt-Dithiolen-Komplexe. Beispiele für im UV-Bereich absorbierende Verbindungen sind Benzotriazole, Triazine, Benzophenone, Cyanoacrylate, Salicylsäurederivate und Hindered Amine Light Stabilizers (HALS). Beispiele für Additive, die im Frequenzbereich der Mikrowellen (1 bis 300 GHz) oder Radiowellen (10 kHz bis 1 GHz) absorbieren, sind ferromagnetische keramische Stoffe, sogenannte Ferrite, die aus den Eisenoxiden Hämatit (Fe₂O₃) oder Magnetit (Fe₃O₄) und weiteren Oxiden beisielweise der Metalle Zn, Mn, oder Ni aufgebaut und als Pulver kommerziell verfügbar sind.

Weiterhin enthalten die erfindungsgemässen Dentalwerkstoffe zur Verbesserung der mechanischen Eigenschaften oder zur Einstellung der Viskosität vorzugsweise auch organische oder anorganische Füllstoffpartikel. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgrösse von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgrösse von 5 bis 200 nm, vorzugsweise 10 bis 100 nm, Minifüllstoffe wie Quarz-, Glaskeramik- oder Glaspulver mit einer durchschnittlichen Teilchengrösse von 0,01 bis 10 µm, vorzugsweise 0,1 bis 1 µm, sowie röntgenopake Füllstoffe wie Ytterbiumtrifluorid oder nanopartikuläres Tantal(V)-oxid bzw. Bariumsulfat mit einer mittleren durchschnittlichen Partikelgrösse von 10 bis 1000 nm, vorzugsweise 100 bis 300 nm.

Ausserdem können die erfindungsgemässen Dentalwerkstoffe weitere Additive enthalten, vor allem Lösungsmittel wie Wasser oder Ethanol bzw. entsprechende Lösungsmittelgemische, sowie beispielsweise Stabilisatoren, Aromastoffe, Farbmittel, mikrobiozide Wirkstoffe, fluoridionenabgebende Additive, optische Aufheller, oder Weichmacher.

Besonders bevorzugt sind Dentalwerkstoffe auf Basis einer thermolabilen polymerisierbaren Verbindung der Formel II, welche die folgenden Bestandteile enthalten:
a) 0,1 bis 50 Gew.-%, insbesondere 1 bis 40 Gew.-%, bevorzugt 2 bis 30 Gew.-% und besonders bevorzugt 5 bis 30 Gew.-% Verbindung der Formel II,
b) 0,01 bis 10 Gew.-%, bevorzugt 0,1 bis 3,0 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff,
f) 0 bis 70 Gew.-% Lösungsmittel.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff.

Dies gilt ebenso für den Lösungsmittelgehalt. Adhäsive enthalten vorzugweise bevorzugt 0 bis 60 Gew.-% und besonders bevorzugt 1 bis 50 Gew.-% Lösungsmittel. Dentalmaterialien, die Wasser als Lösungsmittel enthalten, sind bevorzugt. Besonders bevorzugt sind Dentalmaterialien, die 0 bis 20 Gew.-% und insbesondere 1 bis 10 Gew.-% Wasser enthalten.

Die Debonding-Eigenschaften der erfindungsgemässen Dentalwerkstoffe können durch die Zusammensetzung der Materialien gezielt beeinflusst werden. Die Einstellung einer für einen bestimmten Zweck geeigneten Zusammensetzung gehört zum allgemeinen Wissen und Können des Fachmanns. So nimmt mit der verwendeten Konzentration an thermolabilen Komponenten, d.h. insbesondere der thermolabilen polymerisierbaren Verbindung der Formel II sowie ggf. der thermolabilen Vernetzermonomere und gasfreisetzenden Additive, die Fähigkeit zum gezielten Debonding durch Erwärmen zu. Weiterhin lassen sich die Debonding-Eigenschaften auch durch die Auswahl der Co-Monomere variieren, wobei mit dem Anteil an vernetzenden Monomeren oder durch Zugabe von monofunktionellen Monomeren die Vernetzungsdichte und damit auch die Festigkeit und der E-Modul variiert werden können.

Die erfindungsgemässen Dentalmaterialien auf Basis der thermolabilen polymerisierbaren Verbindung der Formel II können insbesondere zur reversiblen Befestigung beispielsweise von Brackets, Kronen oder Verblendungen (Veneers) verwendet werden. Dabei erfolgt vorzugsweise zunächst die Bildung eines Verbundes durch Aushärtung von Materialien (Adhäsiv oder Zement) auf Basis der thermolabilen polymerisierbaren Verbindung der

Formel II. Zum Debonding müssen die geklebten Teile kurzzeitig auf eine Temperatur erwärmt werden, die über der Temperatur liegt, bei der die Spaltung der thermolabilen Bindungen einsetzt, bzw. mit Licht einer geeigneten Wellenlänge bestrahlt werden. Dabei kann eine gezielte Energiezufuhr beispielsweise über eine IR-Strahlungsquelle oder einen Laser erfolgen. Ausserdem kann beim Einbau von ferromagnetischen Teilchen wie beispielsweise ferromagnetischen Nanopartikeln in die erfindungsgemässen Dentalmaterialien eine induktive Erwärmung durch Einwirkung eines magnetischen Wechselfeldes erreicht werden.

Gegenstand der Erfindung ist auch die Verwendung einer thermolabilen polymerisierbaren Verbindung der Formel II zur Herstellung von Dentalwerkstoffen, vorzugsweise Adhäsiven oder Zementen, besonders bevorzugt selbstätzenden Adhäsiven oder Zementen.

Im Folgenden wird die Erfindung anhand von Ausführungsbeispielen näher erläutert.

### Ausführungsbeispiele

### Beispiel 1

### Synthese von Methacrylsäure-3,5-dioxo-4-(3-phosphonooxy-propyl)-10-oxa-4-aza-tricyclo[5.2.1.0^{2,6}]dec-8-en-1-ylmethylester (MATPA)

### 1. Stufe: 4,10-Dioxa-tricyclo[5.2.1.0^{2,6}]dec-8-en-3,5-dion

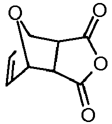

Eine Lösung von Maleinsäureanhydrid (98,06 g, 1,0 mol) und Furan (102,12 g, 1,5 mol) in Acetonitril (200 ml) wurde 96 h bei Raumtemperatur gerührt. Der gebildete Niederschlag wurde abfiltriert, mit Acetonitril (100 ml) gewaschen und im Vakuumtrockenschrank getrocknet (125 mbar, 50 °C). Man erhielt 123,30 g (740 mmol, 74% Ausbeute) eines weissen Feststoffs.
¹H-NMR (DMSO-*d₆*, 400 MHz) : δ = 3,31 (s, 2H), 5,35 (s, 2H), 6,58 (s, 2H).
¹³C-NMR (DMSO-*d₆*, 100 MHz) : δ = 49,0, 81,6, 136,8, 171,5.

### 2. Stufe: 4-(3-Hydroxy-propyl)-10-oxa-4-aza-tricyclo[5.2.1.0^{2,6}]dec-8-en-3,5-dion

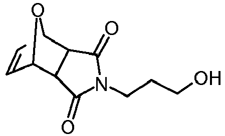

Zu einer Suspension von 4,10-Dioxa-tricyclo[5.2.1.0^{2,6}]dec-8-en-3,5-dion (33,23 g, 200 mmol) in Methanol (70 ml) wurde eine Lösung von 3-Amino-1-propanol (15,02 g, 200 mmol) in Methanol (30 ml) zugetropft. Das Reaktionsgemisch wurde anschliessend am Rückfluss erhitzt. Nach 24 h wurde die Lösung am Rotationsverdampfer eingeengt. Der gelbliche Feststoff wurde in Wasser (100 ml) gelöst und mit Dichlormethan (3x 200 ml) extrahiert. Die vereinigten organischen Phasen wurden über Na₂SO₄ getrocknet, filtriert, am Rotationsverdampfer eingeengt und am Feinvakuum getrocknet. Man erhielt 25,40 g (114 mmol, 57% Ausbeute) eines weissen Feststoffs.
¹H-NMR (DMSO-*d₆*, 400 MHz): δ = 1,59 (m, 2H), 2,91 (s, 2H), 3,38 (m, 4H), 4,45 (br s, 1H), 5,12 (s, 2H), 6,55 (s, 2H).
¹³C-NMR (DMSO-*d₆*, 100 MHz): δ = 30,5, 35,5, 47,0, 58,3, 80,3, 136,4, 176,4.

### 3. Stufe: 1-(3-Hydroxy-propyl)-pyrrol-2,5-dion

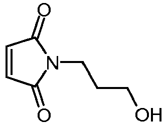

Eine Lösung von 4-(3-Hydroxy-propyl)-10-oxa-4-aza-tricyclo [5.2.1.0^{2,6}]dec-8-en-3,5-dion (17,80 g, 79,8 mmol) in Toluol (300 ml) wurde 16 h am Rückfluss erhitzt. Die Lösung wurde am Rotationsverdampfer eingeengt und der Rückstand am Feinvakuum getrocknet. Man erhielt 11,92 g (76,8 mmol, 96% Ausbeute) eines weissen Feststoffs.
¹H-NMR (DMSO-*d₆*, 400 MHz): δ = 1,65 (m, 2H), 3,40 (t, 2H; *J* = 6,2 Hz), 3,47 (t, 2H; *J* = 7,4 Hz), 4,48 (br s, 1H), 6,99 (s, 2H). ¹³C-NMR (DMSO-*d₆*, 100 MHz): δ = 31,2, 34,7, 58,4, 134,4, 171,0.

### 4. Stufe: Methacrylsäure-4-(3-hydroxypropyl)-3,5-dioxo-10-oxa-4-aza-tricyclo[5.2.1.0^{2,6}]dec-8-en-1-ylmethylester

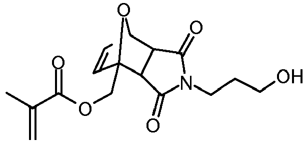

1-(3-Hydroxy-propyl)-pyrrol-2,5-dion (5,17 g, 33,3 mmol), Furfurylmethacrylat (5,65 g, 34,0 mmol) und BHT (10 mg) wurden in Benzol (60 ml) gelöst. Die Lösung wurde unter Einleitung eines leichten Luftstroms am Rückfluss erhitzt. Nach 20 h wurde das Lösungsmittel abdestilliert. Das bräunliche Öl wurde mittels Säulenchromatographie gereinigt (SiO₂, Ethylacetat). Man erhielt 2,84 g (8,8 mmol, 27% Ausbeute, Gemisch aus *exo-* und *endo*-Isomer) eines gelblichen Öls.
¹H-NMR (DMSO-*d₆*, 400 MHz) : δ (*endo*-Isomer) = 1,55 - 1,64 (m, 2H), 1,88 (s, 3H), 3,01 - 3,05 (d, 1H; *J* = 6,4 Hz), 3,09 (d, 1H; *J* = 6,4 Hz), 3,32 - 3,49 (m, 4H), 4,41 (d, 1H; *J* = 12,8 Hz), 4,45 - 4,48 (m, 1H), 4,78 - 4,84 (m, 1H), 5,15 (d, 1H; *J* = 1,5 Hz), 5,68 - 5,70 (m, 1H), 6,00 - 6,03 (m, 1H), 6, 47 - 6,52 (m, 1H), 6,58 - 6,64 (m, 1H).
¹³C-NMR (DMSO-*d₆*, 100 MHz): δ (*endo*-Isomer) = 17,8, 30,5, 35,6, 48,1, 49,6, 58,2, 61,7, 80,5, 88,8, 126,2, 135,4, 136,7, 137,3, 166,1, 174,7, 176,0.

### 5. Stufe: Methacrylsäure-3,5-dioxo-4-(3-phosphonooxy-propyl)-10-oxa-4-aza-tricyclo[5.2.1.0^{2,6}]dec-8-en-1-ylmethylester (MATPA)

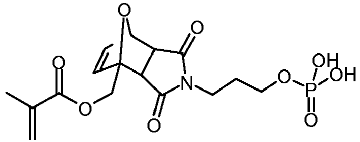

Zu einer Lösung von Phosphoroxychlorid (1,39 g, 9,0 mmol) in Tetrahydrofuran (30 ml) wurde bei -5 °C eine Lösung von Methacrylsäure-4-(3-hydroxypropyl)-3,5-dioxo-10-oxa-4-aza-tricyclo[5.2.1.0^{2,6}]dec-8-en-1-ylmethylester (2,6 g, 8,2 mmol), BHT (10 mg) und Triethylamin (910 mg, 9,0 mmol) in Tetrahydrofuran (20 ml) zugetropft. Nach beendeter Zugabe wurde die Suspension 3 h bei -5 °C gerührt, und dann wurde Wasser (2 ml) zugetropft. Die Suspension wurde weitere 30 min bei -5 °C gerührt, und dann wurde der Niederschlag kalt abfiltriert. Das gelbliche Filtrat wurde mit gesättigter wässriger NaCl-Lösung (3x 30 ml) gewaschen. Die vereinigten wässrigen Phasen wurden mit Tetrahydrofuran (2x 30 ml) reextrahiert. Die vereinigten organischen Phasen wurden über Na₂SO₄ getrocknet, filtriert und am Rotationsverdampfer eingeengt. Das bräunliche Öl wurde zur Wasserentfernung mit Acetonitril (2x 50 ml) versetzt und am Rotationsverdampfer eingeengt. Der Rückstand wurde mit Diethylether (50 ml) versetzt und bei Raumtemperatur gerührt. Nach 1 h wurde das Lösungsmittel abdekantiert. Das braune Öl wurde am Rotationsverdampfer und am Feinvakuum getrocknet. Man erhielt 2,46 g (6,1 mmol, 75% Ausbeute, Gemisch aus *exo-*und *endo*-Isomer) eines bräunlichen Harzes.
¹H-NMR (DMSO-*d₆*, 400 MHz) : δ (*endo*-Isomer) = 1,73 - 1,82 (m, 2H), 1,87 (s, 3H), 3,03 (d, 1H; *J* = 6,5 Hz), 3,10 (d, 1H; *J* = 6,5 Hz), 3,42 - 3,48 (m, 2H), 3,76 - 3,84 (m, 2H), 4,41 (d, 1H; *J* = 12,5 Hz), 4,84 (d, 1H; *J* = 12,5 Hz), 5,15 (s, 1H), 5,69 (s, 1H), 6,01 (s, 1H), 6,50 (d, 1H; *J* = 5,7 Hz), 6,59 - 6,63 (m, 1H), 6,94 (br, 2H) .
¹³C-NMR (DMSO-*d₆*, 100 MHz): δ (*endo*-Isomer) = 17,8, 28,2 (d, *J* = 7 Hz), 35,2, 48,2, 49,7, 61,6, 63,0 (d, *J* = 5 Hz), 80,4, 88,8, 126,2, 135,4, 136,7, 137,3, 166,1, 174,7, 176,0.
³¹P-NMR (DMSO-*d₆*, 162 MHz): δ = -1,3.

### Beispiel 2

### Synthese von Methacrylsäure-3-(3,5-dioxo-1-phosphonooxymethyl-10-oxa-4-aza-tricyclo[5.2.1.0^{2,6}]dec-8-en-4-yl)-propylester

### 1.Stufe: Methacrylsäure-3-(2,5-dioxo-2,5-dihydro-pyrrol-1-yl)-propylester

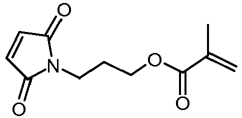

1-(3-Hydroxy-propyl)-pyrrol-2,5-dion (5,36 g, 34,5 mmol), Triethylamin (3,85 g, 38,0 mmol) und *N*,*N*-Dimethylaminopyridin (120 mg, 1,0 mmol) wurden in Dichlormethan (80 ml) gelöst. Eine Lösung von Methacrylsäureanhydrid (5,86 g, 38,0 mmol) und BHT (10 mg) in Dichlormethan (20 ml) wurde bei 0 °C zugetropft, und dann wurde das Reaktionsgemisch 2 h bei 0 °C und 22 h bei Raumtemperatur gerührt. Die Reaktionslösung wurde mit Wasser (3x 50 ml) gewaschen. Die vereinigten wässrigen Phasen wurden mit Dichlormethan (50 ml) reextrahiert. Die vereinigten organischen Phasen wurden über Na₂SO₄ getrocknet, filtriert und am Rotationsverdampfer eingeengt. Das Rohprodukt wurde mittels Säulenchromatographie gereinigt (SiO₂, *n*-Hexan/Ethylacetat 1:1). Man erhielt 2,81 g (12,5 mmol, 35% Ausbeute) eines gelblichen Öls.
¹H-NMR (CDCl₃, 400 MHz) : δ = 1,95 (2, 3H), 1,97-2,04 (m, 2H), 3,66 (t, 2H; *J* = 6,9 Hz), 4,15 (t, 2H; *J* = 6,2 Hz), 5,57 (m, 1H), 6,12 (s, 1H), 6,72 (s, 2H) .
¹³C-NMR (CDCl₃, 100 MHz): δ = 18,3, 27,6, 34,9, 61,8, 125,6, 134,2, 126,2, 167,2, 170,6.

### 2. Stufe: Methacrylsäure-3-(1-hydroxymethyl-3,5-dioxo-10-oxa-4-azatricyclo[5.2.1.0^{2,6}]dec-8-en-4-yl)-propylester

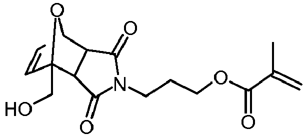

Methacrylsäure-3-(2,5-dioxo-2,5-dihydro-pyrrol-1-yl)-propylester (2,71 g, 12,1 mmol), Furfurylalkohol (1,28 g, 13,0 mmol) und BHT (10 mg) wurden in Benzol (40 ml) gelöst. Die Lösung wurde unter Einleitung eines leichten Luftstroms am Rückfluss erhitzt. Nach 20 h wurde das Lösungsmittel abdestilliert. Das als Rohprodukt erhaltene bräunliche Öl wurde mittels Säulenchromatographie gereinigt (SiO₂, Ethylacetat). Man erhielt 3,10 g (9,6 mmol, 80% Ausbeute, Gemisch aus *exo-* und *endo*-Isomer) als gelbes Öl.
¹H-NMR (CDCl₃, 400 MHz): δ = 1,78-1,89 (m, 0,4H; *exo*), 1,93-2,02 (m, 5,8H; *exo*/*endo*), 2,94-3,02 (m, 3H; *endo*), 3,43-3,48 (m, 0,6H; *exo*), 3,55-3,69 (m, 2,2H; *exo*/*endo*), 4,05-4,14 (m, 4,4H; *exo*/*endo*), 4,15-4,22 (m, 0,2H; *exo*), 4,25-4,31 (m, 0,2H; *exo*), 5,25 (m, 1H; *endo*), 5,28-5,32 (m, 0,2H; *exo*), 5,57-5,60 (m, 1,2H; *exo*/*endo*), 6,10-6,12 (m, 0,2H; *endo*), 6,12-6,14 (m, 1H; *endo*), 6,35-6,38 (m, 0,2H; *exo*), 6,46 - 6,49 (m, 0,2H; *exo*), 6,52-6,56 (m, 1H; *endo*), 6,59-6,62 (m, 1H; *endo*).
¹³C-NMR (CDCl₃, 100 MHz): δ = 18,3, 26,6 *(endo),* 26,7 (*exo*), 35,5 (*exo*), 35,8 (*endo*), 46,0 (*exo*), 48,1 (*endo*), 49,9, 60,7 (*endo*), 61,3 (*exo*), 61,4 (*endo*), 61,6 (*exo*)*,* 79,5 (*exo*)*,* 80,9 (*endo*), 91,5 (*endo*), 92,1 (*exo*), 125,7 (*endo*), 125,8 (*exo*), 134,9 (*exo*), 135,7 (*exo*), 136,1 (*exo*), 136,2 (*endo*), 137,0 (*endo*), 138,3 (*endo*), 167,2 (*exo*)*,* 167,3 (*endo*), 174,7 (*exo*), 175,1 (*exo*), 175,8 (*endo*), 175,9 (*endo*).

### 3. Stufe: Methacrylsäure-3-(3,5-dioxo-1-phosphonooxymethyl-10-oxa-4-aza-tricyclo[5.2.1.0^{2,6}]dec-8-en-4-yl)-propylester

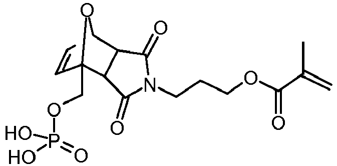

Zu einer Lösung von Phosphoroxychlorid (1,57 g, 10,3 mmol) in Tetrahydrofuran (30 ml) wurde bei -5 °C eine Lösung von Methacrylsäure-3-(1-hydroxymethyl-3,5-dioxo-10-oxa-4-aza-tricyclo[5.2.1.0^{2,6}]dec-8-en-4-yl)-propylester (3,00 g, 9,3 mmol), BHT (10 mg) und Triethylamin (1,04 g, 10,3 mmol) in Tetrahydrofuran (20 ml) zugetropft. Nach beendeter Zugabe wurde die Suspension 3 h bei -5 °C gerührt, und dann wurde Wasser (2 ml) zugetropft. Die Suspension wurde weitere 30 min bei -5 °C gerührt, und dann wurde der Niederschlag kalt abfiltriert. Das gelbliche Filtrat wurde mit gesättigter wässriger NaCl-Lösung (3x 30 ml) gewaschen. Die vereinigten wässrigen Phasen wurden mit Tetrahydrofuran (2x 30 ml) reextrahiert. Die vereinigten organischen Phasen wurden über Na₂SO₄ getrocknet, filtriert und am Rotationsverdampfer eingeengt. Das bräunliche Öl wurde zur Wasserentfernung mit Acetonitril (2x 50 ml) versetzt und am Rotationsverdampfer eingeengt. Der Rückstand wurde mit Diethylether (2x 50 ml) versetzt und bei Raumtemperatur gerührt. Nach 1 h wurde das Lösungsmittel abdekantiert. Das braune Öl wurde am Rotationsverdampfer und am Feinvakuum getrocknet. Man erhielt 3,19 g (7,9 mmol, 85% Ausbeute, Gemisch aus *exo-* und endo-Isomer) eines hygroskopischen weissen Schaums.
¹H-NMR (DMSO-*d₆*, 400 MHz) : δ = 1, 65-1, 75 (m, 0,4H; *exo*), 1,75-1,85 (m, 2H; *endo*), 1,87 (s, 3,6H; *exo*/*endo*), 3,02 (dd, 2H; *J* = 28,2 Hz, 6,4 Hz; *endo*), 3,29 (t, 0,4H; *J* = 7,0 Hz; *exo*), 3,41-3,49 (m, 2,2H; *exo*/*endo*), 3,66 (dd, 0,2H; *J* = 7,8 Hz, 5,6 Hz; *exo*), 3,94-4,08 (m, 3,4H; *exo*/*endo*), 4,32 (dd, 0,2H; *J* = 12,2 Hz, 5,2 Hz; *exo*), 4,42 (dd, 0,2H; *J* = 12,2 Hz, 5,8 Hz; *exo*), 4,54 (q, 1H; *J* = 6,1 Hz; *endo*), 5,08-5,11 (m, 1H; *endo*), 5,25-5,29 (m, 0,2H; *exo*), 5,64-5,68 (m, 1,2H; *exo*/*endo*), 6,00-6,05 (m, 1,2H; *exo*/*endo*), 6,36 (d, 0,2H, *J* = 5,5 Hz; *exo*), 6,44-6,50 (m, 1,2H; *exo*/*endo),* 6,54-6,58 (m, 1H; *endo*), 6,66 (br s, 2,4H; *exo*/*endo*).
¹³C-NMR (DMSO-*d₆*, 100 MHz): δ = 17,9, 26,1 (*exo*), 26,2 (*endo*), 34,6 (*exo*), 34,8 (*endo*), 45,6 (*exo*), 47,3 (*exo*)*,* 48,0 (*endo*), 49,8 (*endo*)*,* 61,5 (*endo*), 61,6 (*exo*), 62,9 (d, *J* = 5 Hz; *endo*), 63,3 (d, *J* = 5 Hz; *exo*), 78,7 (*exo*), 80,4 (*endo*), 89,7 (d, *J* = 10 Hz; *endo*), 90,0 (d, *J* = 10 Hz; *exo*), 125,6, 134,5 (*exo*), 135,4 (*exo*), 135,8 (*endo*), 136,8 (*endo*), 137,0 (*endo*), 166,4, 174,7 (*exo*), 174,7 (*endo*), 174,8 (*exo*), 176,1 (*endo*).

### Beispiel 3

### Radikalische Photopolymerisation des Phosphonsäuremethacrylates MATPA aus Beispiel 1

Es wurde eine Mischung aus 2,97 g des Phosphorsäuremethacrylates MATPA aus Beispiel 1, 6,95 g des Vernetzers N,N'-Diethyl-1,3-bis(acrylamido)-propan, 0,03 g des Photoinitiators Campherchinon und 0,05 g des Aminbeschleunigers 4-(Dimethylamino)-benzoesäureester hergestellt. Ein Tropfen der Mischung wurde auf eine Glasplatte gegeben, mit einer PET-Folie abgedeckt und mit einer Polymerisationslampe Bluephase (Ivoclar Vivadent AG, Lichtintensität 1000 mW/cm⁻²) 20 s lang bestrahlt. Die bestrahlte Schicht war dann ausgehärtet. Ausserdem wurde die Mischung mittels Photo-DSC (Differential Scanning Calorimetry, Perkin Elmer DSC 7) untersucht und ergab eine Polymerisationswärme von 273 J/g.

### Beispiel 4

### Herstellung eines lichthärtenden Adhäsives auf der Basis des Phosphonsäuremethacrylates MATPA aus Beispiel 1

Es wurde ein Adhäsiv aus 1,09 g des Phosphorsäuremethacrylates MATPA aus Beispiel 1, 1,49 g des monofunktionellen Co-Monomers 2-Hydroxyethylmethacrylat, 3,25 g des Vernetzers Bis-GMA, 0,99 g des Vernetzers UDMA, 1,01 g des Vernetzers Glycerindimethacrylat, 0,02 g des Photoinitiators Campherchinon, 0,05 g des Aminbeschleunigers 4-(Dimethylamino)-benzoesäureester, 0,10 g des Acylphosphinoxid-Photoinitiatiors Lucirin TPO und 2,00 g des Lösungsmittels Ethanol hergestellt. Das Adhäsiv konnte mittels einer Polymerisationslampe Bluephase (Ivoclar Vivadent AG, Lichtintensität 1000 mW/cm⁻²) ausgehärtet werden.

### Beispiel 5

### Synthese von Methacrylsäure-3-[3,5-dioxo-1-(11-phosphonooxyundecyl-oxymethyl)-10-oxa-4-aza-tricyclo[5.2.1.0^{2,6}]dec-8-en-4-yl]-propylester

### 1.Stufe: 2-(11-Bromundecyloxy)-tetrahydropyran

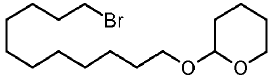

3,4-Dihydro-2H-pyran (21,87 g, 260 mmol) wurde zu einer Lösung von 11-Bromundecanol (50,24 g, 200 mmol) und Toluol-4-sulfonsäure Monohydrat (80 mg, 0,4 mmol) in Dichlormethan (100 ml) zugetropft. Das Reaktionsgemisch wurde bei Raumtemperatur gerührt. Nach 24 h wurde die braune Lösung über eine dünne Schicht Kieselgel filtriert. Das Filtrat wurde am Rotationsverdampfer eingeengt und am Feinvakuum getrocknet. Man erhielt 65,85 g (196 mmol, 98% Ausbeute) eines leicht gelblichen Öls.
¹H-NMR (CDCl₃, 400 MHz): δ = 1,23-1,37 (m, 12H), 1,37-1,47 (m, 2H), 1,48-1,64 (m, 6H), 1,65-1,76 (m, 1H), 1,77-1,90 (m, 3H), 3,35-3,42 (m, 3H), 3,46-3,53 (m, 1H), 3,70-3,75 (m, 1H), 3,84-3,90 (m, 1H), 4,56-4,58 (m, 1H).
¹³C-NMR (CDCl₃, 100,6 MHz): δ = 19,7, 25,4, 26,2, 28,2, 28,8, 29,4, 29,5, 29, 6, 29,8, 30,8, 32,9, 33,9, 62,3, 67,7, 98,8.

### 2.Stufe: 2-[11-(Furan-2-ylmethoxy)-undecyloxy]-tetrahydropyran

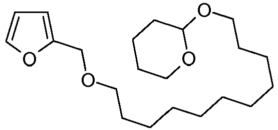

Furfurylalkohol (9,81 g, 100 mmol) wurde zu einer Suspension von Natriumhydrid (2,40 g, 100 mmol) in THF (100 ml) zugetropft. Die Suspension wurde 1 h bei Raumtemperatur gerührt, dann wurde eine Lösung von 2-(11-Bromundecyloxy)-tetrahydropyran (33,53 g, 100 mmol) in THF (100 ml) zugetropft. Das Reaktionsgemisch wurde 16 h am Rückfluss erhitzt. Nach dem Abkühlen wurde mit gesättigter wässriger NH₄Cl-Lösung (100 ml) gequencht. Das Zwei-PhasenGemisch wurde mit Ethylacetat (3x 100 ml) extrahiert. Die vereinigten organischen Phasen wurden über Na₂SO₄ getrocknet, filtriert, am Rotationsverdampfer eingeengt und am Feinvakuum getrocknet. Das Rohprodukt wurde mittels Säulenchromatographie gereinigt (SiO₂, Dichlormethan). Man erhielt 20,58 g (58,4 mmol, 58% Ausbeute) eines gelbliches Öls.
¹H-NMR (CDCl₃, 400 MHz): δ = 1,25-1,40 (m, 16H), 1,48-1,62 (m, 6H), 1,66-1,74 (m, 1H), 1,78-1,87 (m, 1H), 3,34-3,40 (m, 1H), 3,45 (t, 2H; *J* = 6,8 Hz), 3,47-3,52 (m, 1H), 3,70-3,75 (m, 1H), 3,84-3,90 (m, 1H), 4,42 (s, 2H), 4,56-4,58 (m, 1H), 6,28-6,30 (m, 1H), 6,32-6,33 (s, 1H), 7,38-7,39 (m, 1H).
¹³C-NMR (CDCl₃, 100,6 MHz) : δ = 19,7, 25,5, 26,1, 26,3, 29,1, 29,4, 29,5, 29,5, 29,5, 29, 6, 29,7, 29,8, 30,8, 62,3, 64,7, 67,7, 70,4, 98,8, 108,9, 110,2, 142,6, 152,2.

### 3.Stufe: 11-(Furan-2-ylmethoxy)-undecan-1-ol

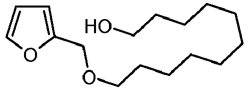

Eine Lösung von 2-[11-(Furan-2-ylmethoxy)-undecyloxy]-tetrahydropyran (20,48 g, 58,1 mmol) und Toluol-4-sulfonsäure Monohydrat (480 mg, 2,4 mmol) in Methanol (100 ml) wurde 20 h bei Raumtemperatur gerührt. Das Reaktionsgemisch wurde am Rotationsverdampfer eingeengt und das Rohprodukt wurde mittels Säulenchromatographie gereinigt (SiO₂, Ethylacetat). Man erhielt 7,18 g (26,8 mmol, 46% Ausbeute) eines gelblichen Feststoffs.
¹H-NMR (CDCl₃, 400 MHz) : δ = 1,25-1,37 (m, 14H), 1,51-1,62 (m, 4H), 1,84 (s, 1H), 3,45 (t, 2H; *J* = 6,8 Hz), 3,61 (t, 2H; *J* = 6,8 Hz), 4,43 (s, 2H), 6,29-6,30 (m, 1H), 6,32-6,34 (m, 1H), 7,39-7,40 (m, 1H).
¹³C-NMR (CDCl₃, 100,6 MHz): δ = 25,8, 26,1, 29,4, 29,5, 29,5, 29, 6, 29, 6, 32,8, 62,9, 64,7, 70,4, 108,9, 110,2, 142,6, 152,1.

### 4.Stufe: Methacrylsäure-3-[3,5-dioxo-1-(11-hydroxy-undecyloxymethyl)-10-oxa-4-aza-tricyclo[5.2.1.0^{2,6}]dec-8-en-4-yl]-propylester

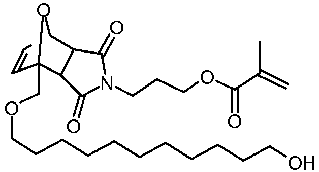

Eine Lösung von 11-(Furan-2-ylmethoxy)-undecan-1-ol (7,00 g, 26,1 mmol), Methacrylsäure-3-(2,5-dioxo-2,5-dihydro-pyrrol-1-yl)-propylester (5,82 g, 26,1 mmol) und BHT (10 mg) in Toluol (100 ml) wurde unter Einleitung eines leichten Luftstroms auf 80 °C erhitzt. Nach 20 h wurde die Lösung am Rotationsverdampfer eingeengt und das Rohprodukt wurde mittels Säulenchromatographie gereinigt (SiO₂, Ethylacetat). Man erhielt 6,16 g (12,5 mmol, 48% Ausbeute, Gemisch aus *exo-* und endo-Isomer) als gelbliches Öl.
¹H-NMR (CDCl₃, 400 MHz) : δ = 1,25-1,36 (m, 22,4H; *exo*/*endo*), 1,51-1,63 (m, 6,6H; *exo*/*endo*), 1,76 (s, 1,6H; *exo*/*endo*), 1,81-1,88 (m, 1,2H; *exo*), 1,92-1,98 (m, 6,4H; *exo*/*endo*), 2,91 (dd, 2H; *J* = 40,2 Hz, 6,4 Hz; *endo*), 3,43-3,51 (m, 2,4H; *exo*), 3,52-3,65 (m, 8,4H), 3,81 (d, 1H; *J* = 11,6 Hz; *endo*), 4,01 (d, 0,6H; *J* = 11,6 Hz; *endo*), 4,06-4,15 (m, 5H; *exo*/*endo*), 5,23-5,24 (m, 1H; *endo*), 5,28-5,30 (m, 0,6H; *exo*), 5,57-5,58 (m, 1,6H; *exo*/*endo*), 6,11-6,14 (m, 1,6H; *exo*/*endo*), 6-30-6,32 (d, 0,6H; *J* = 5,8 Hz; *exo*), 6,44-6,46 (m, 0,6H; *exo*), 6,51-6,54 (m, 2H; *endo*).
¹³C-NMR (CDCl₃, 100,6 MHz): δ = 18,3, 18,3, 25,7, 26,0, 26,7, 29,4, 29,4, 29,4, 29,5, 29,5, 29,5, 29, 6, 32,8, 35,4, 35,7, 45,7, 47,8, 48,3, 49,9, 61,5, 61,6, 62,9, 67,9, 68,4, 72,1, 72,2, 79,6, 81,0, 90,7, 91,4, 125,6, 125,7, 135,1, 135,3, 136,1, 136,2, 136,6, 138,1, 167,2, 174,5, 174,8, 175,0, 176,0.

### 5.Stufe: Methacrylsäure-3-[3,5-dioxo-1-(11-phosphonooxy-undecyloxy-methyl)-10-oxa-4-aza-tricyclo[5.2.1.0^{2,6}]dec-8-en-4-yl]-propylester

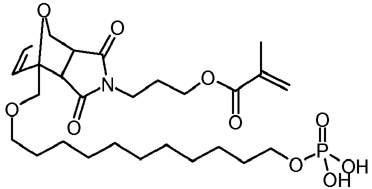

Eine Lösung von Methacrylsäure-3-[3,5-dioxo-1-(11-hydroxy-undecyloxymethyl)-10-oxa-4-aza-tricyclo[5.2.1.0^{2,6}]dec-8-en-4-yl]-propylester (6,06 g, 12,3 mmol), BHT (10 mg) und Triethylamin (1,37 g, 13,6 mmol) in THF (30 ml) wurde bei -5 °C zu einer Lösung von Phosphoroxychlorid (2,08 g, 13,6 mmol) in THF (50 ml) zugetropft. Nach beendeter Zugabe wurde die Suspension 3 h bei - 5 °C gerührt, dann wurde Wasser (2 ml) zugetropft. Die Suspension wurde weitere 30 min im Eisbad gerührt, dann wurde der Niederschlag kalt abfiltriert. Das gelbliche Filtrat wurde mit gesättigter wässriger NaCl-Lösung (3x 50 ml) gewaschen. Die vereinigten wässrigen Phasen wurden mit THF (2x 30 ml) reextrahiert. Die vereinigten organischen Phasen wurden über Na₂SO₄ getrocknet, filtriert und am Rotationsverdampfer eingeengt. Das bräunliche Öl wurde zur Wasserentfernung mit Acetonitril (2x 50 ml) versetzt und am Rotationsverdampfer eingeengt. Das braune Öl wurde mit Diethylether (4x 100 ml) versetzt und 10 min bei Raumtemperatur gerührt. Es schied sich ein dunkelbraunes Öl ab. Das Lösungsmittel wurde abdekantiert. Der vereinigten Ether-Lösung wurden am Rotationsverdampfer eingeengt und am Feinvakuum getrocknet. Man erhielt 3,87 g (6,8 mmol, 55% Ausbeute, Gemisch aus *exo-* und endo-Isomer) eines bräunlichen Öls.
¹H-NMR (DMSO-*d₆*, 400 MHz): δ = 1,22-1,32 (m, 22,4H; *exo*/*endo*), 1,43-1,60 (m, 6,6H; *exo*/*endo*), 1,69-1,76 (m, 1,2H; *exo*), 1,80-1,85 (m, 2H; *endo*), 1,87-1,91 (m, 5,8H; *exo*/*endo*), 2,96 (dd, 2H; *J* = 55,6 Hz, 6,4 Hz; *endo*), 3,30 (t, 1H; *J* = 6,4 Hz; *endo*), 3,36-3,54 (m, 6H; *endo*), 3,59-3,66 (m, 1,6H; *exo*/*endo*), 3,90-4,09 (m, 7,2H; *exo*/*endo*), 5,08-5,09 (m, 1H; *endo*), 5,24-5,26 (m, 0,6H; *exo*), 5,67-5,69 (m, 1,6H; *exo*/*endo*), 6,00-6,06 (m, 1,6H; *exo*/*endo*), 6,32-6,34 (m, 0,6H; *exo*), 6,43-6,47 (m, 1,6H; *exo*/*endo*), 6,52-6,55 (m, 1H; *endo*), 8,68 (br s, 3,2H; *exo*/*endo*)*.*
¹³C-NMR (DMSO-*d₆*, 100,6 MHz): δ = 17,9, 25,1, 25,5, 26,1, 28,6, 28,8, 29,0, 29,0, 29,0, 29,8, 29,9, 30,4, 34,7, 45,5, 47,3, 48,1, 49,6, 61,3, 61,6, 65,2 (d; *J* = 5 Hz), 67,6, 68,1, 70,9, 71,0, 78,7, 80,3, 90,3, 90,8, 125,6, 134,5, 135,9, 135,8, 136,4, 137,6, 166,3, 166,4, 174,7, 174,9, 175,0, 176,2.
³¹P-NMR (DMSO-*d₆*, 162 MHz): δ = -1,1.

### Beispiel 6

### Synthese von Methacrylsäure-3,5-dioxo-4-(10-phosphonooxy-decyl)-10-oxa-4-aza-tricyclo[5.2.1.0^{2,6}]dec-8-en-1-ylmethylester

### 1. Stufe: 4-(10-Hydroxy-decyl)-10-oxa-4-aza-tricyclo[5.2.1.0^{2,6}]dec-8-en-3,5-dion

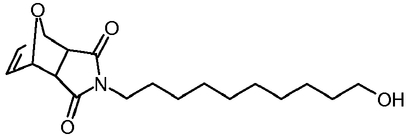

Eine Lösung von 10-Amino-1-decanol (5,36 g, 30,9 mmol) in Methanol (20 ml) wurde zu einer Suspension von 4,10-Dioxatricyclo [5.2.1.0^{2,6}]dec-8-en-3,5-dion (5,13 g, 30,9 mmol) in Methanol (30 ml) zugetropft. Das Reaktionsgemisch wurde 24 h am Rückfluss erhitzt und anschliessend am Rotationsverdampfer eingeengt. Das Rohprodukt wurde mittels Säulenchromatographie gereinigt (SiO₂, Ethylacetat). Man erhielt 1,52 g (4,7 mmol, 15% Ausbeute) eines gelblichen Feststoffs.
¹H-NMR (CDCl₃, 400 MHz): δ = 1,22-1,37 (m, 12H), 1,51-1,59 (m, 4H), 2,60 (br s, 1H), 2,84 (s, 2H), 3,46 (t, 2H; *J* = 7,4 Hz), 3,62 (t, 2H; *J* = 6,5 Hz), 5,26 (s, 2H), 6,51 (s, 2H).
¹³C-NMR (CDCl₃, 100,6 MHz): δ = 25,7, 26,6, 27,5, 29,0, 29,3, 29,3, 29,4, 32,7, 39,0, 47,4, 62,9, 80,9, 136,6, 176,4.

### 2. Stufe: 1-(10-Hydroxy-decyl)-pyrrol-2,5-dion

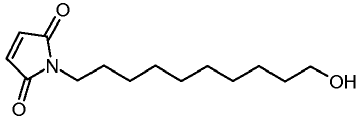

Eine Suspension von 4-(10-Hydroxy-decyl)-10-oxa-4-aza-tricyclo [5.2.1.0^{2,6}]-dec-8-ene-3,5-dion (1,52 g, 4,7 mmol) in Toluol (50 ml) wurde 16 h am Rückfluss erhitzt. Die Lösung wurde vom ungelösten Rückstand abdekantiert, am Rotationsverdampfer eingeengt und am Feinvakuum getrocknet. Man erhielt 1,15 g (4,5 mmol, 97% Ausbeute) eines weissen Feststoffs.
¹H-NMR (CDCl₃, 400 MHz) : δ = 1,25-1,36 (m, 12H), 1,52-1,61 (m, 4H), 2,63 (br s, 1H), 3,50 (t, 2H; *J* = 7,2 Hz), 3,63 (t, 2H; *J* = 6,8 Hz), 6,69 (s, 2H).
¹³C-NMR (CDCl₃, 100,6 MHz) : δ = 25,7, 26,7, 28,5, 29,0, 29,3, 29,4, 32,7, 37,9, 63,0, 134,0, 170,9.

### 3. Stufe: Methacrylsäure-4-(10-hydroxydecyl)-3,5-dioxo-10-oxa-4-aza-tricyclo[5.2.1.0^{2,6}]dec-8-en-1-ylmethylester

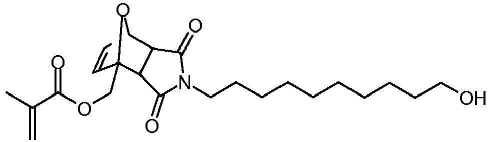

Eine Lösung von Furfurylmethacrylat (750 mg, 4,5 mmol), 1-(10-Hydroxy-decyl)-pyrrol-2,5-dion (1,05 g, 4,1 mmol) und BHT (5 mg) in Toluol (30 ml) wurde unter Einleitung eines leichten Luftstroms auf 80 °C erhitzt. Nach 20 h wurde die Reaktionslösung am Rotationsverdampfer eingeengt und am Feinvakuum getrocknet. Das Rohprodukt wurde säulenchromatographisch gereinigt (SiO₂, *n-*Hexan/Ethylacetat 1:1). Man erhielt 560 mg (1.3 mmol, 33% Ausbeute) des endo-Isomers als weissen Feststoff und 430 mg (1,0 mmol, 25% Ausbeute) des exo-Isomers als gelbliches Öl.

### endo-Isomer:

¹H-NMR (CDCl₃, 400 MHz): δ = 1,19-1,36 (m, 12H), 1,37-1,46 (m, 2H), 1,52-1,61 (m, 3H), 1,97 (s, 3H), 3,31 (t, 2H; *J* = 7,6 Hz), 3,38 (d, 1H; *J* = 7,6 Hz), 3,61-3,66 (m, 3H), 4,69 (d, 1H; *J* = 12,8 Hz), 4,91 (d, 1H; *J* = 12,8 Hz), 5,31 (dd, 1H; *J* = 5,3 Hz, 1,7 Hz), 5,61-5,61 (m, 1H), 6,17 (s, 1H), 6,36 (d, 1H; *J* = 5,8 Hz), 6,45 (dd, 1H; *J* = 5,7 Hz, 1,6 Hz) .
¹³C-NMR (CDCl₃, 100,6 MHz): δ = 18,3, 25, 7, 26,8, 27,4, 29,0, 29,3, 29,4, 32,8, 38,7, 46,8, 47,7, 62,2, 62,9, 79,6, 89,8, 126,5, 134,4, 135,6, 135,7, 166,8, 174,4, 174,6.

### exo-Isomer:

¹H-NMR (CDCl₃, 400 MHz): δ = 1,25-1,35 (m, 12H), 1,52-1,58 (m, 4H), 1,95 (s, 3H), 1,99 (br s, 1H), 2,95 (dd, 2H; *J* = 28,2 Hz, 6,5 Hz), 3,46 (t, 2H; *J* = 7,5 Hz), 3,61 (t, 2H; *J* = 6,8 Hz), 4,52 (d, 1H; *J* = 12,8 Hz), 4,98 (d, 1H; *J* = 12,8 Hz), 5,27 (d, 1H; *J* = 1,6 Hz), 5,59-5,61 (m, 1H), 6,13 (s, 1H), 6,45 (d, 1H; *J* = 5,8 Hz), 6,56 (dd, 1H; *J* = 5,6 Hz, 1,6 Hz).
¹³C-NMR (CDCl₃, 100,6 MHz): δ = 18,3, 25,8, 26,6, 27,5, 29,0, 29,3, 29,3, 29,4, 32,8, 39,0, 48,3, 49,9, 61,6, 62,9, 81,1, 89,6, 126,3, 135,8, 137,1, 137,4, 166,8, 174,3, 175,8.

### 4. Stufe: Methacrylsäure-3,5-dioxo-4-(10-phosphonooxy-decyl)-10-oxa-4-aza-tricyclo[5.2.1.0^{2,6}]dec-8-en-1-ylmethylester

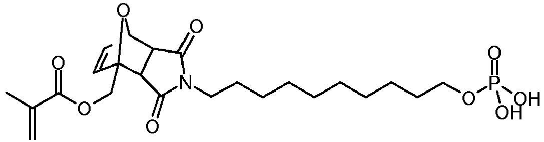

Eine Lösung von endo/exo-Methacrylsäure-4-(10-hydroxydecyl)-3,5-dioxo-10-oxa-4-aza-tricyclo[5.2.1.0^{2,6}]dec-8-en-1-ylmethylester (890 mg, 2,1 mmol), BHT (5 mg) und Triethylamin (240 mg, 2,3 mmol) in THF (30 ml) wurde bei -5 °C zu einer Lösung von Phosphoroxychlorid (360 mg, 2,3 mmol) in THF (20 ml) zugetropft. Nach beendeter Zugabe wurde die Suspension 3 h bei -5 °C gerührt, dann wurde Wasser (2 ml) zugetropft. Die Suspension wurde weitere 30 min im Eisbad gerührt, dann wurde der Niederschlag kalt abfiltriert. Das gelbliche Filtrat wurde mit gesättigter wässriger NaCl-Lösung (3x 50 ml) gewaschen. Die vereinigten wässrigen Phasen wurden mit THF (2x 30 ml) reextrahiert. Die vereinigten organischen Phasen wurden über Na₂SO₄ getrocknet, filtriert und am Rotationsverdampfer eingeengt. Das bräunliche Öl wurde zur Wasserentfernung mit Acetonitril (2x 50 ml) versetzt und am Rotationsverdampfer eingeengt. Der Rückstand wurde am Feinvakuum getrocknet. Man erhielt 1,01 g (2,0 mmol, 95% Ausbeute, Gemisch aus *exo-* und endo-Isomer) eines farblosen Öls.

### Beispiel 7

### Herstellung von DoD-Adhäsiven auf der Basis der Methacrylatphosphate aus Beispiel 5 und 6

Zur Untersuchung der Scherhaftung zwischen einer ZrO₂-Keramik und einem erfindungsgemässen Kompositzement wurden 3 Primerlösungen hergestellt. Dabei handelte es sich um Lösungen von jeweils 1 Gew.-% des Methacrylatphosphats aus Beispiel 5 (Primer A), des Methacrylatphosphats aus Beispiel 6 (Primer B) bzw. von 10-Methacryloyloxydecylphosphat (Primer C, Vergleich) in Ethanol. Zur Bestimmung der Verbundfestigkeit wurden die jeweiligen Primerlösungen auf ZrO₂-Keramikprüfkörper (IPS e.max ZirCAD, Ivoclar Vivadent, Yttrium-stabilisiertes Zirkonoxid) aufgetragen und das Lösungsmittel verblasen. Dann wurde auf die Primerschicht der Kompositzement Multilink Automix (Ivoclar Vivadent) aufgebracht und 20 s mit der LED-Lampe Bluephase C8 (Ivoclar Vivadent) und anschliessend 3 min im Lichtofen Spectramat (Ivoclar Vivadent) ausgehärtet. Anschliessend wurden die Prüfkörper 24 h bei 37 °C in Wasser gelagert und die Scherhaftfestigkeit analog der ISO-Richtlinie "ISO 1994-ISO TR 11405: Dental Materials Guidance on Testing of Adhesion to Tooth Structure" bestimmt. In einer zweiten Serie wurden die Prüfkörper nach Wasserlagerung zusätzlich 60 min bei 130 °C in einem Trockenschrank gelagert und erst dann die Scherhaftung nach schnellem Abkühlen der Prüfkörper bestimmt. Die Ergebnisse sind in Tab. 1 dargestellt und zeigen, dass die Primer mit den erfindungsgemässen Haftmonomeren bei Temperaturbelastung des Keramik-Komposit-Verbundes einen deutlich grösseren Abfall der Verbundfestigkeit aufweisen und sich ein solcher Verbund daher einfacher lösen lässt.

**Tabelle 1:Scherhaftfestigkeit (SH, in MPa) des Verbundes zwischen ZrO₂-Keramik und Kompositzement**

| Primer | SH nach WL^{a)} | SH nach WL+TH^{b)} |
|---|---|---|
| A | 28.4 | 13.9 |
| B | 21.6 | 10.3 |
| C (Vergleich) | 30.1 | 21.7 |

| | | |
|---|---|---|
| ^{a)} WL = Wasserlagerung, ^{b)} WL+TB = Wasserlagerung und thermischer Behandlung | | |

### Beispiel 8

### Synthese von Methacrylsäure-4-[3-(methacryloyloxy)-propyl]-3,5-dioxo-10-oxa-4-aza-tricyclo[5.2.1.0^{2,6}]dec-8-en-1-ylmethylester

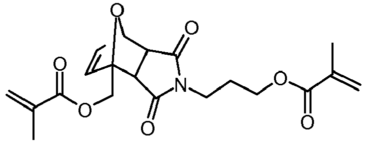

1-Hydroxymethyl-4-(3-hydroxy-propyl)-10-oxa-4-aza-tricyclo[5.2.1.0^{2,6}] dec-8-en-3,5-dion (18,29 g, 72,2 mmol), Triethylamin (16,08 g, 159 mmol), *N*,*N*-Dimethylaminopyridin (600 mg, 5,0 mmol) und BHT (10 mg) wurden in Dichlormethan (100 ml) gelöst. Eine Lösung von Methacrylsäureanhydrid (24,49 g, 159 mmol) in Dichlormethan (50 ml) wurde bei 0 °C zugetropft. Die klare gelbe Lösung wurde 2 h bei -0 °C gerührt, dann wurde das Eisbad entfernt und es wurde bei Raumtemperatur weitergerührt. Nach 22 h wurde die Reaktionslösung mit Wasser (3x 100 ml) gewaschen. Die vereinigten wässrigen Phasen wurden mit Dichlormethan (100 ml) reextrahiert. Die vereinigten organischen Phasen wurden über Na₂SO₄ getrocknet, filtriert und am Rotationsverdampfer eingeengt. Das bräunliche Öl wurde mittels Säulenchromatographie gereinigt (SiO₂, *n-*Hexan/Ethylacetat 2:1). Man erhielt 15,04 g (38,6 mmol, 53% Ausbeute, Gemisch aus *exo-* und *endo*-Isomer) eines gelben Öls.
¹H-NMR (DMSO-*d₆*, 400 MHz): δ = 1,81-1,90 (m, 9,6H; *exo*/*endo*), 3,04 (d, 1H; *J* = 6,5 Hz; *endo*), 3,10 (d, 1H; *J* = 6,5 Hz; *endo*), 3,31-3,35 (m, 0,4H; *exo*), 3,47-3,51 (m, 2,2H; *exo*/*endo*), 3,70-3,73 (m, 0,2H; *exo*), 3,99-4,05 (m, 2,4H; *exo*/*endo*), 4,41 (d, 1H; *J* = 12,6 Hz; *endo*), 4,63 (d, 0,2H; *J* = 12,8 Hz; *exo*), 4,79 (d, 0,2H; *J* = 12,8 Hz; *exo*), 4,84 (d, 1H; *J* = 12,6 Hz; *endo*), 5,15 (d, 1H; *J* = 1,6 Hz; *endo*), 5,32 (dd, 0,2H; *J* = 5,6 Hz, 1,5 Hz; *exo*), 5,67-5,69 (m, 2,2H; *exo*/*endo*), 5,71-5,73 (m, 0,2H; *exo*), 6,01-6,08 (m, 2,4H; *exo*/*endo*), 6,42 (d, 0,2H; *J* = 6,1 Hz; *exo*), 6,49-6,53 (m, 1,2H; *exo*/*endo*), 6,60-6,62 (m, 1H; *endo*)*.*
¹³C-NMR (DMSO-*d₆*, 100,6 MHz): δ = 17,8 *(endo),* (17,8; *exo*), 17,9, (26,2), 26,2, (34,6), 34,9, (46,4), (47,3), 48,2, 49,7, 61,4, (61,5), 61,6, (62,0), (78,8), 80,5, 88,8 (89,1), 125,5, 126,1, (126,3), (134,3), (135,4), 135,5, (135,7), 135,8, 136,6, 137,2, 166,0, 166,4, (174,5), (174,6), 174,7, 176,0.

### Beispiel 9

### Synthese von Methacrylsäure-3,5-dioxo-4-(3-triethoxysilylpropyl)-10-oxa-4-aza-tricyclo[5.2.1.0^{2,6}]dec-8-en-1-ylmethylester

### 1. Stufe: N-(3-Triethoxysilylpropyl)maleimid (SI126)

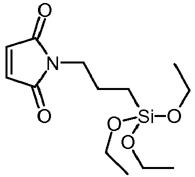

Maleinsäureanhydrid (29,42 g, 300 mmol) wurde in Toluol (100 ml) suspendiert. Eine Lösung von 3-Aminopropyltriethoxysilan (66,41 g, 300 mmol) in Toluol (75 ml) wurde zugetropft und die Reaktionslösung wurde bei Raumtemperatur gerührt. Nach 2 h wurde zunächst Zinkchlorid (13,63 g, 100 mmol) zugegeben, dann wurde eine Lösung von Hexamethyldisilazan (60,52 g, 375 mmol) in Toluol (75 ml) zugetropft. Die Suspension wurde 24 h am Rückfluss erhitzt und nach dem Abkühlen auf Raumtemperatur über Celatom filtriert. Das Filtrat wurde am Rotationsverdampfer eingeengt und am Feinvakuum getrocknet. Das Rohprodukt wurde durch Vakuumdestillation gereinigt (Kp: 125 °C/0,03 mbar). Man erhielt 15,14 g (50,3 mmol, 17% Ausbeute) einer farblosen Flüssigkeit.
¹H-NMR (CDCl₃, 400 MHz): δ = 0,41-0,49 (m, 2H), 1,08 (t, 9H; *J* = 7,1 Hz), 1,49-1,60 (m, 2H), 3,37 (t, 2H; *J* = 7,3 Hz), 3,60-3,70 (m, 6H), 6,59 (s, 2H).
¹³C-NMR (CDCl₃, 100,6 MHz): δ = 6,1, 16,7, 20,5, 38,7, 56,8, 132,5, 169,2.
²⁹Si-NMR (CDCl₃, 79,5 MHz): δ = -46,4.

### 2. Stufe: Methacrylsäure-3,5-dioxo-4-(3-triethoxysilylpropyl)-10-oxa-4-aza-tricyclo[5.2.1.0^{2,6}]dec-8-en-1-ylmethylester

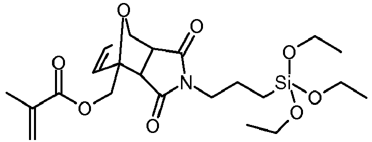

Eine Lösung von Furfurylmethacrylat (8,24 g, 49,6 mmol), *N*-(3-Triethoxysilylpropyl)-maleimid (14,94 g, 49,6 mmol) und BHT (10 mg) in Toluol (150 ml) wurde unter Einleitung eines leichten Luftstroms auf 80 °C erhitzt. Nach 20 h wurde das Lösungsmittel am Rotationsverdampfer und das Rohprodukt wurde mittels Säulenchromatographie gereinigt (SiO₂, *n*-Hexan/Ethylacetat 2:1). Man erhielt 5,67 g (12,1 mmol, 24% Ausbeute, Gemisch aus *exo-* und endo-Isomer) eines farblosen Öls.
¹H-NMR (CDCl₃, 400 MHz): δ = 0,43-0,47 (m, 2,2H; *exo*/*endo*), 1,06-1,10 (m, 9,9H; *exo*/*endo*), 1,37-1,46 (m, 0,2H; *exo*), 1,50-1,58 (m, 2H, *endo*), 1,82 (s, 3H; *endo*), 1,84 (s, 0,3H; *exo*), 2,85 (dd, 2H, *J* = 28,2 Hz, 6,4 Hz; *endo*), 3,19 (t, 0,2H; *J* = 7,3 Hz; *exo*), 3,28 (d, 0,2H; *J* = 7,3 Hz; *exo*), 3,35 (t, 2H; *J* = 7,3 Hz; *endo*), 3,63-3,71 (m, 6, 6H; *exo*/*endo*), 4,38 (d, 1H, *J* = 12,7 Hz; *endo*), 4,56 (d, 0,1H, *J* = 12,7 Hz; *exo*), 4,78 (d, 0,1H, *J* = 12,7 Hz; *exo*), 4,87 (d, 1H, *J* = 12,7 Hz; *endo*), 5,15 (s, 1H; *endo*), 5,18-5,20 (m, 0,1H; *exo*), 5,48 (s, 1H; *endo*), 5,50 (s, 0,1H; *exo*), 6,00 (s, 1H; *endo*), 6,05 (s, 0,1H; *exo*), 6,22 (d, 0,1H, *J* = 5,4 Hz; *exo*), 6,34 (d, 1, 1H, *J* = 5,4 Hz; *endo*/*exo*), 6,45 - 6,47 (m, 1H; *endo*).
¹³C-NMR (CDCl₃, 100,6 MHz): δ = 5,9 (*endo*), 6,3 (*exo*), 16,6, 19,5, 45,2 (*exo*), 46,1 (*exo*), 46,7 (*endo*), 48,3 (*endo*), 56,7, 60,0 *(endo),* 60,6 (*exo*), 77,9 (*exo*), 79,4 *(endo),* 87,9 (*endo*), 78,1 (*exo*), 124,6 *(endo),* 124,8 (*exo*), 132,8 (*exo*), 134,0 (*exo*), 134,1 (*exo*), 134,1 *(endo),* 135,5 *(endo),* 135,8 *(endo),* 165,1, 172,7 *(endo),* 172,8 (*exo*), 172,9 (*exo*), 174,2 *(endo).*
²⁹Si-NMR (CDCl₃, 79,5 MHz): δ = -46,6 (*exo*), -46,3 (*endo*).

## Patentansprüche

1. Dentalwerkstoff, der eine thermolabile polymerisierbare Verbindung der Formel II enthält: 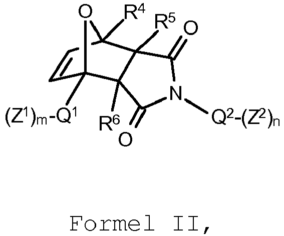 in der
Z¹ und Z² jeweils unabhängig für eine polymerisierbare Gruppe ausgewählt aus Vinylgruppen, CH₂=CR¹-CO-O- und CH₂=CR¹-CO-NR²- oder für eine adhäsive Gruppe ausgewählt aus -Si(OR)₃, -COOH, -O-PO(OH)₂, -PO(OH)₂, -SO₂OH und -SH stehen, wobei mindestens ein Z¹ oder Z² eine polymerisierbare Gruppe ist,
Q¹ jeweils unabhängig entfällt oder für einen (m+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³-unterbrochen sein kann,
Q² jeweils unabhängig entfällt oder für einen (n+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³-unterbrochen sein kann,
R, R¹, R² und R³ jeweils unabhängig für H oder einen C₁-C₇-Alkyl-Rest stehen und
R⁴ H oder ein C₁-C₁₀-Alkyl-Rest ist,
R⁵ H, ein C₁-C₅-Alkyl-Rest, F oder CN ist,
R⁶ H, ein C₁-C₅-Alkyl-Rest, F oder CN ist,
m und n jeweils unabhängig 1, 2 oder 3 sind.

2. Dentalwerkstoff nach Anspruch 1, bei dem jeweils unabhängig voneinander
eines von Z¹ und Z² jeweils unabhängig für eine polymerisierbare Gruppe ausgewählt aus CH₂=CR¹⁻CO-O- und CH₂=CR¹-CO-NR²- steht und das andere von Z¹ und Z² jeweils unabhängig für eine polymerisierbare Gruppe ausgewählt aus CH₂=CR¹-CO-O- und CH₂=CR¹-CO-NR²- oder vorzugsweise für eine adhäsive Gruppe ausgewählt aus -Si(OR)₃, -COOH, -O-PO(OH)₂, -PO(OH)₂, SO₂OH und -SH steht,
Q¹ jeweils unabhängig entfällt oder für einen (m+1)-wertigen linearen oder verzweigten aliphatischen C₁-C₁₅-Rest, vorzugsweise einen C₁-C₁₀-Rest, bevorzugt einen C₁-C₈-Rest, insbesondere einen C₂-C₆-Rest und besonders bevorzugt einen C₁-C₂-Rest steht, der durch -O-, -CO-O-, -O-CO-, -CO-NR³-, -NR³-CO-, -O-CO-NR³-, -NR³-CO-O- oder -NR³-CO-NR³- unterbrochen sein kann,
Q² jeweils unabhängig entfällt oder für einen (n+1)-wertigen linearen oder verzweigten aliphatischen C₁-C₁₅-Rest, vorzugsweise einen C₁-C₁₀-Rest, bevorzugt einen C₁-C₈-Rest, insbesondere einen C₂-C₆-Rest und besonders bevorzugt einen C₂-C₃-Rest steht, der durch -O-, -CO-O-, -O-CO-, -CO-NR³-, -NR³-CO-, -O-CO-NR³-, -NR³-CO-O- oder -NR³-CO-NR³- unterbrochen sein kann,
R jeweils unabhängig CH₃ oder C₂H₅ ist,
R¹ jeweils unabhängig H oder CH₃ ist,
R² jeweils unabhängig H, CH₃ oder C₂H₅ ist,
R³ jeweils unabhängig H, CH₃ oder C₂H₅ ist,
R⁴ H, CH₃ oder C₂H₅ ist,
R⁵ H, F oder CN und insbesondere H ist,
R⁶ H, F oder CN und insbesondere H ist und/oder
m und n jeweils unabhängig 1 oder 2 sind.

3. Dentalwerkstoff nach einem der vorhergehenden Ansprüche, der ein oder mehrere zusätzliche radikalisch polymerisierbare Monomere enthält.

4. Dentalwerkstoff nach Anspruch 3, der
Methyl-, Ethyl-, Hydroxyethyl-, Butyl-, Benzyl-, Tetrahydrofurfuryl- oder Isobornyl(meth)acrylat, Bisphenol-A-di(meth) acrylat, Bis-GMA, UDMA, Di-, Tri- oder Tetraethylenglycol-di(meth)acrylat, Trimethylolpropantri(meth)acrylat, Pentaerythrittetra(meth)acrylat, Glycerindi(meth)acrylat, 1,4-Butandioldi(meth)acrylat, 1,10-Decandioldi(meth)acrylat, 1,12-Dodecandioldi(meth)acrylat,
und/oder
ein oder mehrere N-mono- oder -disubstitiuierte Acrylamide, N-Ethylacrylamid, N,N-Dimethacrylamid, N-(2-Hydroxyethyl)-acrylamid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether,
und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acryl-amido)-propan, 1,3-Bis-(methacrylamido)-propan, 1,4-Bis-(acrylamido)-butan, 1,4-Bis(acryloyl)-piperazin,
und/oder
ein oder mehrere thermolabile Vernetzermonomere oder eine Mischung davon enthält.

5. Dentalwerkstoff nach einem der vorhergehenden Ansprüche, der ein oder mehrere thermolabile Vernetzermonomere enthält.

6. Dentalwerkstoff nach einem der vorhergehenden Ansprüche, der ein oder mehrere radikalisch polymerisierbare, säuregruppenhaltige Monomere enthält.

7. Dentalwerkstoff nach Anspruch 6, der
Maleinsäure, Acrylsäure, Methacrylsäure, 2-(Hydroxymethyl)-acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin, 4-Vinylbenzoesäure,
und/oder
Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methylpentyl-phosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester,
und/oder
2-Methacryloyloxypropylmono- oder -dihydrogenphosphat, 2-Methacryloyloxyethylphenyl-hydrogenphosphat, Dipentaerythritolpentamethacryloyloxyphosphat, 10-Methacryloyloxydecyldihydrogenphosphat, Phosphorsäuremono-(1-acryloyl-piperidin-4-yl)-ester, 6-(Methacrylamido)hexyldihydrogenphosphat, 1,3-Bis-(N-acryloyl-N-propyl-amino)-propan-2-yldihydrogenphosphat,
und/oder
Vinylsulfonsäure, 4-Vinylphenylsulfonsäure, 3-(Methacryl-amido)-propylsulfonsäure,
oder eine Mischung davon enthält.

8. Dentalwerkstoff nach einem der vorhergehenden Ansprüche, der einen Initiator für die radikalische Polymerisation enthält.

9. Dentalwerkstoff nach einem der vorhergehenden Ansprüche, der ein thermisch gasfreisetzendes Additiv enthält.

10. Dentalwerkstoff nach einem der vorhergehenden Ansprüche, der ein Additiv enthält, das eingestrahlte elektromagnetische Strahlung in Wärme umwandeln kann.

11. Dentalwerkstoff nach einem der vorhergehenden Ansprüche, der organischen und/oder anorganischen Füllstoff enthält.

12. Dentalwerkstoff nach einem der vorhergehenden Ansprüche, der
a) 0,1 bis 50 Gew.-%, insbesondere 1 bis 40 Gew.-%, bevorzugt 2 bis 30 Gew.-% und besonders bevorzugt 5 bis 30 Gew.-% Verbindung der Formel II,
b) 0,01 bis 10 Gew.-%, bevorzugt 0,1 bis 3,0 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff,
f) 0 bis 70 Gew.-% Lösungsmittel
enthält.

13. Dentalwerkstoff nach Anspruch 11 oder 12 zur Verwendung als Adhäsiv, der 0 bis 20 Gew.-% Füllstoff enthält.

14. Dentalwerkstoff nach Anspruch 11 oder 12 zur Verwendung als Komposit, der 20 bis 80 Gew.-% Füllstoff enthält.

15. Verwendung einer Verbindung der Formel II wie in einem der Ansprüche 1 und 2 definiert zur Herstellung eines Dentalwerkstoffs, vorzugsweise eines Adhäsivs oder Zements und insbesondere eines selbstätzenden Adhäsivs oder Zements.

## Claims

1. Dental material comprising a thermolabile polymerisable compound of Formula II: 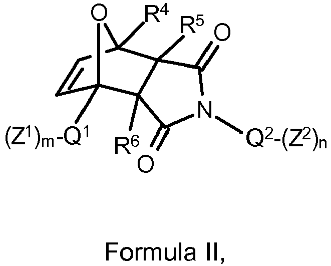 in which
Z¹ and Z² each independently stand for a polymerisable group selected from vinyl groups, CH₂=CR¹-CO-O- and CH₂=CR¹-CO-NR²- or for an adhesive group selected from -Si(OR)₃, -COOH, -O-PO(OH)₂, - PO(OH)₂, -SO₂OH and -SH, wherein at least one Z¹ or Z² is a polymerisable group,
Q¹ each independently is missing or stands for an (m+1)-valent linear or branched aliphatic C₁-C₂₀ group which can be interrupted by -O-, -S-, -CO-O-, -O-CO-, -CO-NR³- or -NR³-CO-, -O-CO-NR³-, -NR³-CO-O- or -NR³-CO-NR³-,
Q² each independently is missing or stands for an (n+1)-valent linear or branched aliphatic C₁-C₂₀ group that can be interrupted by -O-, -S-, - CO-O-, -O-CO-, -CO-NR³-, -NR³-CO-, -O-CO-NR³-, -NR³-CO-O- or - NR³-CO-NR³-,
R, R¹, R² and R³ each independently stand for H or a C₁-C₇ alkyl group and
R⁴ is H or a C₁-C₁₀ alkyl group
R⁵ is H, a C₁-C₅ alkyl group, F or CN,
R⁶ is H, a C₁-C₅ alkyl group, F or CN,
m and n are each independently 1, 2 or 3.

2. Dental material according to claim 1, in which each independently of one another
one of Z¹ and Z² each independently stands for a polymerisable group selected from CH₂=CR¹-CO-O- and CH₂=CR¹-CO-NR²- and the other Z¹ and Z² each independently stands for a polymerisable group selected from CH₂=CR¹-CO-O- and CH₂=CR¹-CO-NR²- or preferably stands for an adhesive group selected from -Si(OR)₃, - COOH, -O-PO(OH)₂, -PO(OH)₂, -SO₂OH and -SH,
Q¹ each independently is missing or stands for an (m+1)-valent linear or branched aliphatic C₁-C₁₅ group, preferably a C₁-C₁₀ group, preferably a C₁-C₈ group, in particular a C₂-C₆ group and particularly preferably a C₁-C₂ group, which can be interrupted by - O-, -CO-O-, -O-CO-, -CO-NR³-, -NR³-CO-, -O-CO-NR³-, -NR³-CO-O- or -NR³-CO-NR³-,
Q² each independently is missing or stands for an (n+1)-valent linear or branched aliphatic C₁-C₁₅ group, preferably a C₁-C₁₀ group, preferably a C₁-C₈ group, in particular a C₂-C₆ group and particularly preferably a C₁-C₂ group, which can be interrupted by - O-, -CO-O-, -O-CO-, -CO-NR³-, -NR³-CO-, -O-CO-NR³-, -NR³-CO-O- or -NR³-CO-NR³-,
R each independently is CH₃ or C₂H₅,
R¹ each independently is H or CH₃,
R² each independently is H, CH₃ or C₂H₅,
R³ each independently is H, CH₃ or C₂H₅,
R⁴ is H, CH₃ or C₂H₅,
R⁵ is H, F or CN and in particular H,
R⁶ is H, F or CN and in particular H and/or
m and n each independently are 1 or 2.

3. Dental material according to one of the preceding claims, which comprises one or more additional radically polymerisable monomers.

4. Dental material according to claim 3, which comprises methyl, ethyl, hydroxyethyl, butyl, benzyl, tetrahydrofurfuryl or *iso*bornyl (meth)acrylate, bisphenol-A-di(meth)acrylate, bis-GMA, UDMA, di-, tri- or tetraethylene glycol di(meth)acrylate, trimethylolpropane tri(meth)acrylate, pentaerythritol tetra(meth)acrylate, glycerol di(meth)acrylate, 1,4-butanediol di(meth)acrylate, 1,10-decanediol di(meth)acrylate, 1,12-dodecanediol di(meth)acrylate,
and/or
one or more *N*-mono- or disubstituted acrylamides, *N*-ethylacrylamide, *N,N*-dimethacrylamide, *N*-(2-hydroxyethyl)acrylamide, *N*-methyl-*N*-(2-hydroxyethyl)acrylamide, one or more *N*-monosubstituted methacrylamides, *N*-ethylmethacrylamide, *N*-(2-hydroxyethyl)methacrylamide, *N*-vinyl pyrrolidone, one or more cross-linking allyl ethers,
and/or
one or more cross-linking pyrrolidones, 1,6-bis(3-vinyl-2-pyrrolidonyl)-hexane, one or more cross-linking bisacrylamides, methylene or ethylene bisacrylamide, one or more cross-linking bis(meth)acrylamides, *N,N*'-diethyl-1,3-bis(acrylamido)-propane, 1,3-bis(methacrylamido)-propane, 1,4-bis(acrylamido)-butane, 1,4-bis(acryloyl)-piperazine,
and/or
one or more thermolabile cross-linking monomers
or a mixture thereof.

5. Dental material according to one of the preceding claims, which comprises one or more thermolabile cross-linking monomers.

6. Dental material according to one of the preceding claims, which comprises one or more radically polymerisable, acid group-containing monomers.

7. Dental material according to claim 6, which comprises maleic acid, acrylic acid, methacrylic acid, 2-(hydroxymethyl)acrylic acid, 4-(meth)acryloyloxyethyltrimellitic anhydride, 10-methacryloyloxydecylmalonic acid, *N*-(2-hydroxy-3-methacryloyloxypropyl)-*N*-phenylglycine, 4-vinylbenzoic acid,
and/or
vinylphosphonic acid, 4-vinylphenylphosphonic acid, 4-vinyl-benzylphosphonic acid, 2-methacryloyloxyethylphosphonic acid, 2-methacrylamidoethylphosphonic acid, 4-methacrylamido-4-methyl-pentyl-phosphonic acid, 2-[4-(dihydroxyphosphoryl)-2-oxa-butyl]-acrylic acid, ethyl or 2,4,6-trimethylphenyl esters of 2-[4-(dihydroxyphosphoryl)-2-oxa-butyl]-acrylic acid
and/or
2-methacryloyloxypropyl mono- or dihydrogen phosphate, 2-methacryloyloxyethylphenyl hydrogen phosphate, dipentaerythritol-pentamethacryloyloxy phosphate, 10-methacryloyloxydecyl dihydrogen phosphate, phosphoric acid mono-(1-acryloyl-piperidin-4-yl) ester, 6-(methacrylamido)hexyl dihydrogen phosphate, 1,3-bis-(*N*-acryloyl-*N-*propylamino)-propan-2-yl dihydrogen phosphate,
and/or
vinylsulfonic acid, 4-vinylphenylsulfonic acid, 3-(methacryl-amido)propylsulfonic acid,
or a mixture thereof.

8. Dental material according to one of the preceding claims, which comprises an initiator for radical polymerisation.

9. Dental material according to one of the preceding claims, which comprises an additive that when heated releases gas.

10. Dental material according to one of the preceding claims, which comprises an additive that can convert radiated electromagnetic radiation into heat.

11. Dental material according to one of the preceding claims, which comprises organic and/or inorganic filler.

12. Dental material according to one of the preceding claims, which comprises
a) 0.1 to 50 wt %, in particular 1 to 40 wt %, preferably 2 to 30 wt % and particularly preferably 5 to 30 wt % compound of Formula II,
b) 0.01 to 10 wt %, preferably 0.1 to 3.0 wt % and particularly preferably 0.2 to 2 wt % initiator,
c) 0 to 80 wt %, preferably 1 to 60 wt % and particularly preferably 5 to 50 wt % comonomer,
d) 0 to 30 wt %, preferably 0.5 to 15 wt % and particularly preferably 1 to 5 wt % adhesive monomer,
e) 0 to 80 wt % filler,
f) 0 to 70 wt % solvent.

13. Dental material according to claim 11 or 12 for use as an adhesive, which comprises 0 to 20 wt % filler.

14. Dental material according to claim 11 or 12 for use as a composite, which comprises 20 to 80 wt % filler.

15. Use of a compound of Formula II as defined in one of claims 1 and 2, for the preparation of a dental material, preferably an adhesive or cement and in particular a self-etching adhesive or cement.

## Revendications

1. Matériau dentaire qui contient un composé polymérisable thermolabile de formule II : 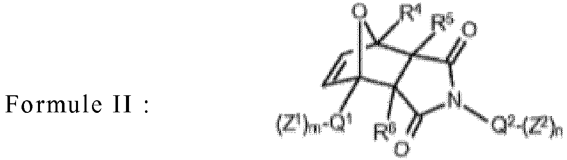 dans laquelle
- Z¹ et Z² représentent, indépendamment en chaque occurrence, un groupe polymérisable, choisi parmi le groupe vinyle et les groupes de formule CH₂=CR¹-CO-O- ou CH₂=CR¹-CO-NR²-, ou un groupe promoteur d'adhésion, choisi parmi ceux de formule -Si(OR)₃, -COOH, -PO(OH)₂, -O-PO(OH)₂, -SO₂OH ou -SH, étant entendu qu'au moins l'un des symboles Z¹ et Z² représente un groupe polymérisable,
- Q¹, indépendamment, ne représente rien ou représente un groupe aliphatique en C₁-C₂₀, linéaire ou ramifié, de valence m+1, au sein duquel peut être intercalé un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- Q², indépendamment, ne représente rien ou représente un groupe aliphatique en C₁-C₂₀, linéaire ou ramifié, de valence n+1, au sein duquel peut être intercalé un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- R, R¹, R² et R³ représentent, indépendamment en chaque occurrence, un atome d'hydrogène ou un groupe alkyle en C₁-C₇,
- R⁴ représente un atome d'hydrogène ou un groupe alkyle en C₁-C₁₀,
- R⁵ représente un atome d'hydrogène, un groupe alkyle en C₁-C₅, un atome de fluor ou un groupe cyano,
- R⁶ représente un atome d'hydrogène, un groupe alkyle en C₁-C₅, un atome de fluor ou un groupe cyano,
- et les indices m et n valent chacun, indépendamment, 1, 2 ou 3.

2. Matériau dentaire conforme à la revendication 1, dans lequel, indépendamment les uns des autres,
- l'un des symboles Z¹ et Z² représente, indépendamment en chaque occurrence, un groupe polymérisable choisi parmi les groupes de formule CH₂=CR¹-CO-O- ou CH₂=CR¹-CO-NR²-, et l'autre des symboles Z¹ et Z² représente, indépendamment en chaque occurrence, un groupe polymérisable choisi parmi les groupes de formule CH₂=CR¹-CO-O- ou CH₂=CR¹-CO-NR²-, ou, de préférence, un groupe promoteur d'adhésion choisi parmi ceux de formule -Si(OR)₃, -COOH, -O-PO(OH)₂, -PO(OH)₂, -SO₂OH ou -SH,
- Q¹, indépendamment, ne représente rien ou représente un groupe aliphatique linéaire ou ramifié, de valence m+1, en C₁-C₁₅, de préférence en C₁-C₁₀, mieux encore en C₁-C₈ et en particulier en C₂-C₆, mais surtout en C₁-C₂, au sein duquel peut être intercalé un chaînon ou un raccord symbolisé par -O-, -CO-O-, -O-CO-, -CO-NR³-, -NR³-CO-, -O-CO-NR³-, -NR³-CO-O- ou -NR³-CO-NR³-,
- Q², indépendamment, ne représente rien ou représente un groupe aliphatique linéaire ou ramifié, de valence n+1, en C₁-C₁₅, de préférence en C₁-C₁₀, mieux encore en C₁-C₈ et en particulier en C₂-C₆, mais surtout en C₂-C₃, au sein duquel peut être intercalé un chaînon ou un raccord symbolisé par -O-, -CO-O-, -O-CO-, -CO-NR³-, -NR³-CO-, -O-CO-NR³-, -NR³-CO-O- ou -NR³-CO-NR³-,
- R représente, indépendamment en chaque occurrence, un groupe méthyle ou éthyle,
- R¹ représente, indépendamment en chaque occurrence, un atome d'hydrogène ou un groupe méthyle,
- R² représente, indépendamment en chaque occurrence, un atome d'hydrogène ou un groupe méthyle ou éthyle,
- R³ représente, indépendamment en chaque occurrence, un atome d'hydrogène ou un groupe méthyle ou éthyle,
- R⁴ représente un atome d'hydrogène ou un groupe méthyle ou éthyle,
- R⁵ représente un atome d'hydrogène, un atome de fluor ou un groupe cyano, mais en particulier un atome d'hydrogène,
- R⁶ représente un atome d'hydrogène, un atome de fluor ou un groupe cyano, mais en particulier un atome d'hydrogène,
- et/ou les indices m et n valent chacun, indépendamment, 1 ou 2.

3. Matériau dentaire conforme à l'une des revendications précédentes, qui contient un ou plusieurs monomère(s) polymérisable(s) par voie radicalaire supplémentaire(s).

4. Matériau dentaire conforme à la revendication 3, qui contient
- de l'acrylate ou du méthacrylate de méthyle, d'éthyle, d'hydroxy-éthyle, de butyle, de benzyle, de tétrahydrofuryle ou d'isobornyle, du di(méth)acrylate de bisphénol A, du bis-GMA, de l'UDMA, du di(méth)acrylate de diéthylèneglycol, de triéthylèneglycol ou de tétraéthylèneglycol, du tri(méth)acrylate de triméthylolpropane, du tétra(méth)acrylate de pentaérythritol, du di(méth)acrylate de glycérol, du di(méth)acrylate de butane-1,4-diol, du di(méth)acrylate de décane-1,10-diol, du di(méth)acrylate de dodécane-1,12-diol,
- et/ou un ou plusieurs acrylamide(s) portant un ou deux substituant(s) placé(s) sur l'atome d'azote, N-éthyl-acrylamide, N,N-diméthyl-acrylamide, N-(2-hydroxy-éthyl)-acrylamide, N-méthyl-N-(2-hydroxy-éthyl)-acrylamide, un ou plusieurs méthacrylamide(s) portant un seul substituant placé sur l'atome d'azote, N-éthyl-méthacrylamide, N-(2-hydroxy-éthyl)-méthacrylamide, de la N-vinyl-pyrrolidone, un ou plusieurs éther(s) d'allyle promoteur(s) de réticulation,
- et/ou une ou plusieurs pyrrolidone(s) promotrice(s) de réticulation, 1,6-bis(3-vinyl-2-pyrrolidonyl)-hexane, un ou plusieurs bis-acrylamide(s) promoteur(s) de réticulation, méthylène-bis-acrylamide, éthylène-bis-acrylamide, un ou plusieurs bis-(méth)acrylamide(s) promoteur(s) de réticulation, N,N'-diéthyl-1,3-bis(acrylamido)-propane, 1,3-bis(méthacrylamido)-propane, 1,4-bis(acrylamido)-butane, de la 1,4-bis(acryloyl)-pipérazine,
- et/ou un ou plusieurs monomère(s) thermolabile(s) promoteur(s) de réticulation,
ou un mélange de tels monomères.

5. Matériau dentaire conforme à l'une des revendications précédentes, qui contient un ou plusieurs monomère(s) thermolabile(s) promoteur(s) de réticulation.

6. Matériau dentaire conforme à l'une des revendications précédentes, qui contient un ou plusieurs monomère(s) polymérisable(s) par voie radicalaire et comportant un groupe acide.

7. Matériau conforme à la revendication 6, qui contient
- de l'acide maléique, de l'acide acrylique, de l'acide méthacrylique, de l'acide 2-(hydroxy-méthyl)-acrylique, de l'anhydride 4-((méth)-acryloyloxy-éthyl)-trimellique, de l'acide (10-méthacryloyloxy-décyl)-malonique, de la N-(2-hydroxy-3-méthacryloyloxy-propyl)-N-phényl-glycine, de l'acide 4-vinyl-benzoïque,
- et/ou de l'acide vinyl-phosphonique, de l'acide 4-vinyl-phényl-phosphonique, de l'acide 4-vinyl-benzyl-phosphonique, de l'acide 2-méthacryloyloxy-éthyl-phosphonique, de l'acide 2-méthacrylamido-éthyl-phosphonique, de l'acide 4-méthacrylamido-4-méthyl-pentyl-phosphonique, de l'acide 2-[4-(dihydroxyphosphoryl)-2-oxa-butyl]-acrylique, du 2-[4-(dihydroxyphosphoryl)-2-oxa-butyl]-acrylate d'éthyle ou de 2,4,6-triméthyl-phényle,
- et/ou du monohydrogénophosphate ou dihydrogénophosphate de 2-méthacryloyloxy-propyle, de l'hydrogénophosphate de phényle et de 2-méthacryloyloxy-éthyle, du phosphate de penta(méthacryloyloxy)-dipentaérythritol, du dihydrogénophosphate de 10-méthacryloyloxy-décyle, du phosphate de mono(1-acryloyl-pipéridin-4-yle), du dihydrogénophosphate de 6-méthacrylamido-hexyle, du dihydrogénophosphate de 1,3-bis(N-acryloyl-N-propyl-amino)-prop-2-yle,
- et/ou de l'acide vinyl-sulfonique, de l'acide 4-vinyl-benzènesulfonique, de l'acide 3-méthacrylamido-propanesulfonique,
ou un mélange de ces composés.

8. Matériau dentaire conforme à l'une des revendications précédentes, qui contient un amorceur de polymérisation par voie radicalaire.

9. Matériau dentaire conforme à l'une des revendications précédentes, qui contient un adjuvant qui peut libérer un gaz sous l'effet de la chaleur.

10. Matériau dentaire conforme à l'une des revendications précédentes, qui contient un adjuvant qui peut convertir en chaleur un rayonnement électromagnétique incident.

11. Matériau dentaire conforme à l'une des revendications précédentes, qui contient une charge organique et/ou une charge inorganique.

12. Matériau dentaire conforme à l'une des revendications précédentes, qui contient :
a) de 0,1 à 50 % en poids, en particulier de 1 à 40 % en poids, de préférence de 2 à 30 % en poids, et mieux encore de 5 à 30 % en poids, d'un composé de formule II,
b) de 0,01 à 10 % en poids, de préférence de 0,1 à 3,0 % en poids, et mieux encore de 0,2 à 2 % en poids, d'un amorceur,
c) de 0 à 80 % en poids, de préférence de 1 à 60 % en poids, et mieux encore de 5 à 50 % en poids, d'un co-monomère,
d) de 0 à 30 % en poids, de préférence de 0,5 à 15 % en poids, et mieux encore de 1 à 5 % en poids, d'un monomère promoteur d'adhésion,
e) de 0 à 80 % en poids d'une charge,
d) et de 0 à 70 % en poids d'un solvant.

13. Matériau dentaire conforme à la revendication 11 ou 12 pour utilisation en tant qu'adhésif, qui contient de 0 à 20 % en poids d'une charge.

14. Matériau dentaire conforme à la revendication 11 ou 12 pour utilisation en tant que matériau composite, qui contient de 20 à 80 % en poids d'une charge.

15. Utilisation d'un composé de formule II, tel que défini dans l'une des revendications 1 et 2, pour la préparation d'un matériau dentaire qui est de préférence un adhésif ou un ciment, et en particulier, un adhésif ou ciment auto-mordant.
